(19) Europäisches Patentamt
European Patent Office
Office européen des brevets

(11) EP 2 376 482 B1

(12) EUROPÄISCHE PATENTSCHRIFT

(45) Veröffentlichungstag und Bekanntmachung des Hinweises auf die Patenterteilung:
**06.03.2013 Patentblatt 2013/10**

(21) Anmeldenummer: **09796960.4**

(22) Anmeldetag: **15.12.2009**

(51) Int Cl.:
***C07D 409/06*** $^{(2006.01)}$ ***A61P 31/12*** $^{(2006.01)}$
***A61K 31/4178*** $^{(2006.01)}$

(86) Internationale Anmeldenummer:
**PCT/EP2009/008992**

(87) Internationale Veröffentlichungsnummer:
**WO 2010/075962 (08.07.2010 Gazette 2010/27)**

(54) **SUBSTITUIERTE (THIOPHENYL-CARBONYL)IMIDAZOLIDINONE UND IHRE VERWENDUNG**
SUBSTITUTED (THIOPHENYL-CARBONYL)IMIDAZOLIDINONES, AND USE THEREOF
(THIOPHÉNYLCARBONYL)IMIDAZOLIDINONES À SUBSTITUTION ET LEUR UTILISATION

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **17.12.2008 DE 102008062863**

(43) Veröffentlichungstag der Anmeldung:
**19.10.2011 Patentblatt 2011/42**

(73) Patentinhaber: **AiCuris GmbH & Co. KG**
**42117 Wuppertal (DE)**

(72) Erfinder:
- **THEDE, Kai**
  **10405 Berlin (DE)**
- **GRESCHAT, Susanne**
  **49419 Wagenfeld (DE)**
- **WILDUM, Steffen**
  **58332 Schwelm (DE)**
- **PAULSEN, Daniela**
  **42105 Wuppertal (DE)**

(74) Vertreter: **Witte, Weller & Partner**
**Phoenixbau**
**Königstrasse 5**
**70173 Stuttgart (DE)**

(56) Entgegenhaltungen:
**WO-A1-2009/115213 WO-A2-2009/115252**

- **ROMERO D L ET AL: "discovery,synthesis and bioactivity of bis(heteroaryl)piperazines.1.A Novel Class of Non-Nucleoside HIV-1 Reverse Transcriptase Inhibitors" JOURNAL OF MEDICINAL CHEMISTRY, AMERICAN CHEMICAL SOCIETY, WASHINGTON, US, Bd. 37, Nr. 7, 1. Januar 1994 (1994-01-01) , Seiten 999-1014, XP002345620 ISSN: 0022-2623**

EP 2 376 482 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft neue substituierte (Thiophenyl-carbonyl)imidazolidinone, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von retroviralen Erkrankungen, bei Menschen und/oder Tieren.

HIV (Virus der humanen Immundefizienz) verursacht eine chronisch-persistente, progrediente Infektion. Die Erkrankung verläuft über verschiedene Stadien von der asymptomatischen Infektion bis zum Krankheitsbild AIDS (*Acquired Immunodeficiency Syndrom*). AIDS ist das finale Stadium der durch Infektion hervorgerufenen Erkrankung. Charakteristisch für die HIV/AIDS-Erkrankung ist die lange klinische Latenzzeit mit persistierender Virämie, die im Endstadium zum Versagen der Immunabwehr führt.

Durch die Einführung der anti-HIV Kombinationstherapie gelang es in den 90-er Jahren, die Krankheitsprogression nachhaltig zu verlangsamen und damit die Lebenserwartung HIV-infizierter Patienten substantiell zu verlängern (Palella et al., N. Engl. J. Med. 1998, 238, 853-860).

Die derzeit auf dem Markt befindlichen anti-HIV-Substanzen hemmen die Replikation des HI-Virus durch Inhibition der essentiellen viralen Enzyme Reverse Transkriptase (RT), Protease oder Integrase oder des Eintritts von HIV in die Zielzelle (Übersicht in Flexner, Nature Reviews Drug Discovery 2007, 6, 959-966). Es existieren zwei Klassen von RT-Inhibitoren: Nukleosidische und nukleotidische RT-Inhibitoren (NRTI) wirken durch kompetitive Inhibition oder Kettenabbruch bei der DNA-Polymerisation. Nicht-nukleosidische RT-Inhibitoren (NNRTI) binden allosterisch an einer hydrophoben Tasche in der Nähe des aktiven Zentrums der RT und vermitteln eine Konformationsänderung des Enzyms. Die derzeit verfügbaren Proteaseinhibitoren (PI) blockieren das aktive Zentrum der viralen Protease und verhindern somit die Reifung neuentstehender Partikel zu infektiösen Virionen. Der einzige derzeit zugelassene Integrase-Inhibitor Raltegravir bindet in das aktive Zentrum der HIV-Integrase und verhindert die Integration der proviralen DNA in das Wirtszellgenom. "Entry-Inhibitoren" (Fusions-Inhibitoren und Korezeptor-Antagonisten) verhindern die HIV-Infektion von Zellen durch Interaktion mit dem HIV-Hüllprotein oder durch Blockierung der zellulären Korezeptoren CCR5 oder CXCR4.

Da die Monotherapie mit den momentan verfügbaren anti-HIV-Medikamenten innerhalb sehr kurzer Zeit zum Therapieversagen durch Selektion resistenter Viren führt, erfolgt üblicherweise eine Kombinationstherapie mit mehreren anti-HIV-Substanzen aus verschiedenen Klassen (*highly active antiretroviral therapy* = HAART; Carpenter et al., J. Am. Med. Assoc. 2000, 283, 381-390).

Trotz der Fortschritte in der antiretroviralen Chemotherapie zeigen neuere Untersuchungen, dass mit den zur Verfügung stehenden Medikamenten eine Eradikation von HIV und damit verbunden eine Heilung der HIV-Infektion nicht zu erwarten ist. Das latente Virus verbleibt in ruhenden Lymphozyten und stellt ein Reservoir für eine Reaktivierung und damit für eine erneute Virusausbreitung dar (Finzi et al., Nature Med. 1999, 5, 512-517; Ramratnam et al., Nature Med. 2000, 6, 82-85). HIVinfizierte Patienten sind daher zeitlebens auf eine effiziente antivirale Therapie angewiesen. Trotz Kombinationstherapie kommt es nach einiger Zeit zur Selektion resistenter Viren. Da für jede therapeutische Klasse charakteristische Resistenzmutationen akkumulieren, bedeutet das Versagen einer Therapie oft einen Wirkverlust der kompletten Substanzklasse. Diese Kreuzresistenzproblematik ist bei der Klasse der NNRTIs am ausgeprägtesten, da hier oft schon eine einzelne Punktmutation in der RT ausreichen kann, um einen Wirkverlust aller NNRTIs zu bewirken (Übersicht in Kavlick & Mitsuya, Antiretroviral Chemotherapy (Hrsg. De Clercq E.), 2001, ASM Press, 279-312).

Begünstigt wird die Entstehung von Resistenzen meist durch die schlechte Compliance der Patienten, die durch ein ungünstiges Nebenwirkungsprofil und kompliziertes Dosierungsschema der anti-HIV-Medikamente hervorgerufen wird.

Somit besteht ein dringender Bedarf nach neuen therapeutischen Optionen zur Bekämpfung der HIV-Infektion. Dazu ist es ein vordringliches Ziel der Therapieforschung zu HIV, neue chemische Leitstrukturen zu identifizieren, die entweder ein neues Target in der Vermehrung des HIV adressieren und/oder gegen die wachsende Zahl resistenter klinischer HIV-Isolate wirksam sind.

Die WO 91/19708 A1 offenbart Thiophencarboxamide mit schmerzstillenden und antiinflammatorischen Wirkungen. Die EP 0 065 295 A1 offenbart Thiophencarboxamide zur Behandlung von Herz- und Kreislauferkrankungen. Die WO 94/27979 A1 offenbart Thiophencarboxamide als Modulatoren von Peptiden vom Endothelin-Typ. In der WO 02/00649 A1, der WO 2006/062982 A2 und der WO 2006/062984 A2 sind Thiophencarboxamide als Inhibitoren von verschiedenen Kinasen beschrieben. Aus der US 5,627,203 und der WO 03/014107 A1 sind gewisse Thiophencarboxamide als Zwischenstufe in der Synthese von polyzyklischen Verbindungen bekannt. Aus der US 2004/0116425 A1 bzw. der WO 2004/016592 A1 sind Thiophencarboxamide als Inhibitoren der Proteinprenylierung bekannt. In der WO 2005/035488 A2 sind Thiophencarboxamide als Antagonisten des Cannabinoid-Rezeptors $CB_1$ beschrieben und in der WO 2006/023462 A1 als Antagonisten des Histamin-H3-Rezeptors. Aus der WO 2008/0903820 A1 sind Thiophencarboxamide zur Behandlung von durch Prionen hervorgerufener Krankheiten, Krebs und Erkrankungen des zentralen Nervensystems sowie zur Regulierung von Stammzellen bekannt.

Romero et al., J. Med. Chem 1994, 37, 999-1014 offenbart Piperazinderivate zur Behandlung *von* retroviralen Erkrankungen.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen, die die zuvor beschriebenen Nachteile nicht aufweisen.

Überraschenderweise wurde gefunden, dass die in der vorliegenden Erfindung beschriebenen substituierten (Thiophenyl-carbonyl)imidazolidinone antiviral wirksam sind.

Gegenstand der Erfindung sind Verbindungen der Formel

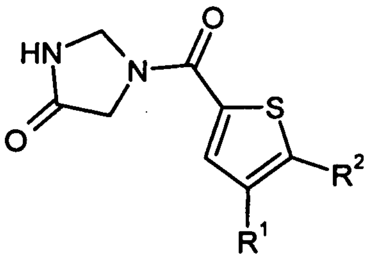

(I),

in welcher

$R^1$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,
worin
$(C_1-C_4)$-Alkyl und $(C_1-C_4)$-Alkoxy ihrerseits ein- bis dreifach, gleich oder verschieden, mit Resten substituiert sein können, die ausgewählt werden aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können, und

$R^2$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,
worin
$(C_1-C_4)$-Alkyl und $(C_1-C_4)$-Alkoxy ihrerseits ein- bis dreifach, gleich oder verschieden, mit Resten substituiert sein können, die ausgewählt werden aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können,

und ihre Salze, ihre Solvate und die Solvate ihrer Salze.

Erfindungsgemäße Verbindungen sind die Verbindungen der Formeln (I) und (Ia) und deren Salze, Solvate und Solvate der Salze, sowie die von den Formeln (I) und (Ia) umfassten, nachfolgend als Ausführungsbeispiel(e) genannten Verbindungen und deren Salze, Solvate und Solvate der Salze, soweit es sich bei den von den Formeln (I) und (Ia) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht

geeignet sind, aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als <u>Solvate</u> werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl sowie die Alkylteile in Alkoxy und Alkoxycarbonyl stehen für geradkettiges oder verzweigtes Alkyl und umfassen, wenn nicht anders angegeben, ($C_1$-$C_6$)-Alkyl, insbesondere ($C_1$-$C_4$)-Alkyl, wie z.B. Methyl, Ethyl, Propyl, Isopropyl, Butyl, Isobutyl.

Alkoxy steht im Rahmen der Erfindung vorzugsweise für einen geradkettigen oder verzweigten Alkoxyrest insbesondere mit 1 bis 6, 1 bis 4 bzw. 1 bis 3 Kohlenstoffatomen. Bevorzugt ist ein geradkettiger oder verzweigter Alkoxyrest mit 1 bis 3 Kohlenstoffatomen. Beispielhaft und vorzugsweise seien genannt: Methoxy, Ethoxy, n-Propoxy, Isopropoxy, t-Butoxy, n-Pentoxy und n-Hexoxy.

<u>Alkoxycarbonyl</u> steht beispielhaft und vorzugsweise für Methoxycarbonyl, Ethoxycarbonyl, n-Propoxycarbonyl, Isopropoxycarbonyl, t-Butoxycarbonyl, n-Pentoxycarbonyl und n-Hexoxycarbonyl.

<u>Heterocyclyl</u> steht für einen monocyclischen, heterocyclischen Rest mit 4 bis 7, vorzugsweise 5 bis 6 Ringatomen und bis zu 3, vorzugsweise bis zu 2 Heteroatomen und/oder Heterogruppen aus der Reihe N, O, S, SO, $SO_2$, wobei ein Stickstoffatom auch ein N-Oxid bilden kann. Der Heterocyclus kann gesättigt oder teilweise ungesättigt sein. Bevorzugt sind 5- bis 7-gliedrige, monocyclische gesättigte Heterocyclen mit bis zu zwei Heteroatomen aus der Reihe O, N und S, beispielhaft und vorzugsweise 1,4-Oxazepanyl, Oxetan-3-yl, Pyrrolidin-1-yl, Pyrrolidin-2-yl, Pyrrolidin-3-yl, Tetrahydrofuranyl, Tetrahydrothienyl, Pyranyl, 1,3-Thiazolidinyl, Piperidin-1-yl, Piperidin-2-yl, Piperidin-3-yl, Piperidin-4-yl, Thiopyranyl, Morpholin-2-yl, Morpholin-3-yl, Morpholin-4-yl, Thiomorpholin-2-yl, Thiomorpholin-3-yl, Thiomorpholin-4-yl, Perhydroazepinyl, Piperazin-1-yl, Piperazin-2-yl.

<u>Halogen</u> steht für Fluor, Chlor, Brom oder Jod, wobei Fluor und Chlor bevorzugt sind, wenn nichts anderes angegeben ist.

<u>Mono-($C_1$-$C_4$)-alkylamino</u> steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n*-Butylamino, *tert*.-Butylamino, *n*-Pentyl-amino und *n*-Hexylamino.

<u>Di-($C_1$-$C_4$)-alkylamino</u> steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N*-Diethylamino, *N*-Ethyl-*N*-methylamino, *N*-Methyl-*N*-*n*-propylamino, N-Isopropyl-*N*-*n*-propylamino, *N,N*-Diisopropylamino, *N*-*n*-Butyl-*N*-methylamino, N-tert.-Butyl-*N*-methylamino, N-Methyl-*N*-*n*-pentylamino und *N*-*n*-Hexyl-N-methylamino.

<u>($C_3$-$C_7$)-Cycloalkyl</u> steht im Rahmen der Erfindung für einen monocyclischen, gesättigten Carbocyclus mit 3 bis 7 bzw. 3 bis 6 Ring-Kohlenstoffatomen. Beispielhaft und vorzugsweise seien genannt: Cyclopropyl, Cyclobutyl, Cyclopentyl, Cyclohexyl und Cycloheptyl.

Die oben aufgeführten allgemeinen oder in Vorzugsbereichen angegebenen Restedefinitionen gelten sowohl für die Endprodukte der Formeln (I) und (Ia) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen angegebenen Restedefinitionen werden unabhängig von den jeweilig angegebenen Kombinationen der Reste beliebig auch durch Restedefinitionen anderer Kombinationen ersetzt.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1\text{-}C_4)$-Alkyl und $(C_1\text{-}C_4)$-Alkoxy, und

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1\text{-}C_4)$-Alkyl und $(C_1\text{-}C_4)$-Alkoxy,
worin
$C_1\text{-}C_4$-Alkoxy seinerseits ein- bis dreifach, gleich oder verschieden, mit Resten substituiert sein kann, die ausgewählt werden aus der Reihe Halogen, Cyano, Hydroxy, $(C_1\text{-}C_4)$-Alkoxy, Amino, Mono-$(C_1\text{-}C_4)$-alkylamino, Di-$(C_1\text{-}C_4)$-alkylamino, $(C_3\text{-}C_7)$-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, $(C_1\text{-}C_4)$-Alkyl, Trifluormethyl, Hydroxy, $(C_1\text{-}C_4)$-Alkoxy, Trifluormethoxy, Oxo, Amino, Mono-$(C_1\text{-}C_4)$-alkylamino und Di-$(C_1\text{-}C_4)$-alkylamino substituiert sein können,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl, Methyl und Methoxy, und

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl, Trifluormethoxy, Methyl und $(C_1\text{-}C_3)$-Alkoxy,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano, und

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano und Trifluormethyl,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano, und

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano

und ihre Salze, ihre Solvate und die Solvate ihrer Salze.

Gegenstand der Erfindung sind auch Verbindungen der Formel

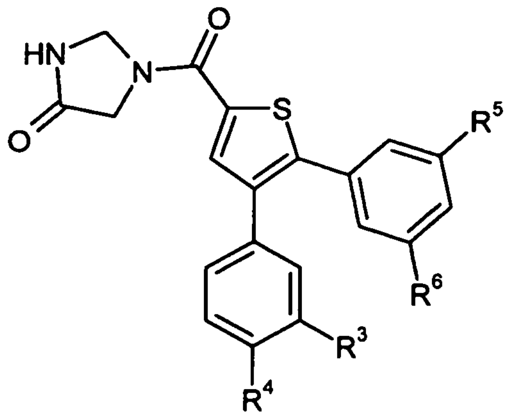

(Ia),

in welcher

$R^3$ für Halogen oder Cyano steht,

$R^4$ für Wasserstoff oder Halogen steht,

$R^5$ für Halogen, Cyano oder Trifluormethyl steht, und

$R^6$ für Wasserstoff oder Halogen steht,

und ihre Salze, ihre Solvate und die Solvate ihrer Salze.

Gegenstand der Erfindung sind auch Verbindungen der Formel (Ia), in welcher

$R^3$ für Halogen oder Cyano steht,

$R^4$ für Wasserstoff oder Halogen steht,

$R^5$ für Halogen oder Cyano steht, und

$R^6$ für Wasserstoff oder Halogen steht,

und ihre Salze, ihre Solvate und die Solvate ihrer Salze.

Gegenstand der Erfindung sind auch Verbindungen der Formel (Ia), in welcher

$R^3$ für Fluor, Chlor oder Cyano steht,

$R^4$ für Wasserstoff, Chlor oder Fluor steht,

$R^5$ für Fluor, Chlor oder Cyano steht, und

$R^6$ für Wasserstoff, Chlor oder Fluor steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,

$R^4$ für Wasserstoff oder Fluor steht,

$R^5$ für Chlor oder Cyano steht, und

$R^6$ für Wasserstoff oder Fluor steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,

$R^4$ für Fluor steht,

$R^5$ für Chlor oder Cyano steht, und

$R^6$ für Fluor steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,

$R^4$ für Fluor steht,

$R^5$ für Chlor oder Cyano steht, und

$R^6$ für Wasserstoff steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,

$R^4$ für Wasserstoff steht,

$R^5$ für Chlor oder Cyano steht, und

$R^6$ für Wasserstoff steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,

$R^4$ für Wasserstoff steht,

$R^5$ für Chlor oder Cyano steht, und

$R^6$ für Fluor steht,

und ihre Salze, ihre Solvate und die Solvate ihrer Salze.

Gegenstand der Erfindung ist weiterhin ein Verfahren zur Herstellung der Verbindungen der Formeln (I) und (Ia), wobei Verbindungen der Formel

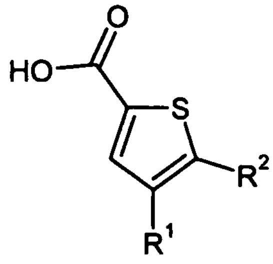

(II),

in welcher
$R^1$ und $R^2$ die oben angegebene Bedeutung aufweisen,
mit Imidazolidin-4-on oder einem Salz von Imidazolidin-4-on umgesetzt werden.

Die Umsetzung erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoffe wie Benzol oder Toluol, Nitromethan, Tetrahydrofuran, 1,4-Dioxan, Dimethylformamid oder Acetonitril. Ebenso ist es möglich, Gemische der Lösemittel einzusetzen. Besonders bevorzugt sind Dichlormethan, Dimethylformamid, Tetrahydrofuran oder Toluol.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Als Dehydratisierungsreagenzien eignen sich hierbei beispielsweise Carbodiimide wie z.B. *N,N'*-Diethyl-, *N,N,'*-Dipropyl-, *N,N'*-Diisopropyl-, *N,N'*-Dicyclohexylcarbodiimid, *N*-(3-Dimethylaminoisopropyl)-*N'*-ethylcarbodiimid-hydrochlorid (EDC), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methylisoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)phosphorylchlorid, oder *O*-(Benzotriazol-1-yl)-*N,N,N',N'*-tetramethyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N,N,N',N'*-tetramethyluroniumhexafluorophosphat (HATU), oder 1-Hydroxybenzotriazol (HOBt), oder Benzotriazol-1-yloxytris(dimethylamino)phosphoniumhexafluorophosphat (BOP), oder Benzotriazol-1-yloxytris(pyrrolidino)phosphoniumhexafluorophosphat (PyBOP), oder *N*-Hydroxysuccinimid, oder Mischungen aus diesen, mit Basen.

Vorzugsweise wird die Kondensation mit PyBOP, TBTU oder mit EDC in Gegenwart von HOBt durchgeführt.

In einem alternativen Verfahren können die Verbindungen der Formel (II) zunächst mit Thionylchlorid und in der zweiten Stufe mit Imidazolidin-4-on oder einem Salz von Imidazolidin-4-on in Gegenwart einer Base, wie z. B. Triethylamin, umgesetzt werden.

Die nach den oben angegebenen Verfahren hergestellten Verbindungen der Formel (I) und (Ia) tragen gegebenenfalls Schutzgruppen, die nach dem Fachmann bekannten Bedingungen abgespalten werden können, um weitere Verbindungen der Formel (I) und (Ia) zu erhalten.

Die Verbindungen der Formel (II) sind bekannt oder können hergestellt werden, indem in Verbindungen der Formel

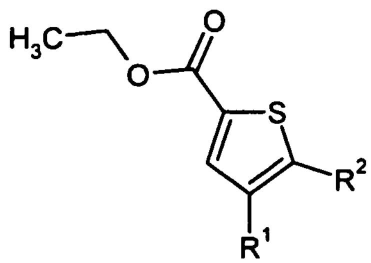

(III),

in welcher
$R^1$ und $R^2$ die oben angegebene Bedeutung aufweisen,
der Ester mit einer Base verseift wird.

Die Verseifung des Esters mit einer Base erfolgt im Allgemeinen in inerten Lösungsmitteln, bevorzugt in einem

Temperaturbereich von Raumtemperatur bis zum Rückfluss des Lösungsmittels bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt sind Lithium-, Kalium- oder Natriumhydroxid.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-*tert*-butylether, 1,2-Dimethoxyethan, 1,4-Dioxan, Tetrahydrofuran, Glykoldimethylether oder Diethylenglykoldimethylether, Alkohole wie Methanol, Ethanol, n-Propanol, iso-Propanol, n-Butanol oder *tert*-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Wasser, oder Gemische von Lösungsmitteln. Als Lösungsmittel sind bevorzugt 1,4-Dioxan, Tetrahydrofuran und/oder Methanol. Bevorzugt ist Lithiumhydroxid in Tetrahydrofuran - oder 1,4-Dioxan-Wasser-Gemischen oder Kaliumhydroxid in Methanol.

Die Verbindungen der Formel (III) sind bekannt oder können hergestellt werden, indem Verbindungen der Formel

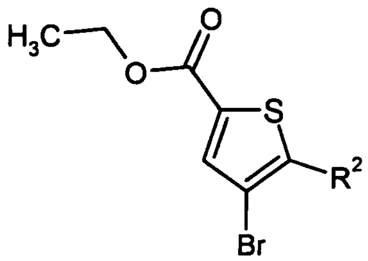

(IV),

in welcher

$R^2$ die oben angegebene Bedeutung aufweist,

unter Suzuki-Kupplungsbedingungen mit Verbindungen der Formel

$R^1$-Q (V),

in welcher

$R^1$ die oben angegebene Bedeutung aufweist und

Q für $-B(OH)_2$, einen Boronsäure-Ester, bevorzugt Boronsäurepinakolester, oder $-BF_3^-K^+$ steht,

umgesetzt werden.

Die Suzuki-Kupplungen erfolgen im Allgemeinen in inerten Lösungsmitteln, in Gegenwart eines Katalysators, gegebenenfalls in Gegenwart eines Zusatzreagenzes, bevorzugt in einem Temperaturbereich von Raumtemperatur bis 130°C bei Normaldruck.

Katalysatoren sind beispielsweise für Suzuki-Reaktionsbedingungen übliche Palladium-Katalysatoren, bevorzugt sind Katalysatoren wie z.B. Dichlorbis(triphenylphosphin)palladium, Tetrakistriphenylphosphinpalladium(0), Palladium(II)acetat, Palladium(II)acetat/Triscyclohexylphosphin oder Bis(diphenylphosphanferrocenyl)-palladium-(II)-chlorid oder Palladium(II)acetat mit einem Liganden wie Dicyclohexyl[2',4',6'-tri(propan-2-yl)biphenyl-2-yl]phosphan.

Zusatzreagenzien sind beispielsweise Kaliumacetat, Cäsium-, Kalium- oder Natriumcarbonat, Kalium-tert.-butylat, Cäsiumfluorid oder Kaliumphosphat, bevorzugt sind Zusatzreagenzien wie z.B. Kaliumacetat und/oder eine wässrige Natriumcarbonatlösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon, oder Gemische der Lösungsmittel mit Alkoholen wie Methanol oder Ethanol und/oder Wasser, bevorzugt ist 1,2-Dimethoxyethan.

Die Verbindungen der Formel (V) sind bekannt oder lassen sich nach bekannten Verfahren aus den entsprechenden Edukten synthetisieren.

Die Verbindungen der Formel (IV) sind bekannt oder können hergestellt werden, indem die Verbindung der Formel

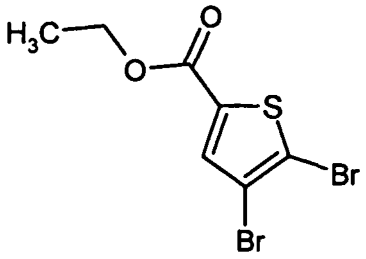

(VI),

unter den oben angegebenen Suzuki-Kupplungsbedingungen mit Verbindungen der Formel

$R^2$-Q (VII),

in welcher
$R^2$ die oben angegebene Bedeutung aufweist und

Q für $-B(OH)_2$, einen Boronsäure-Ester, bevorzugt Boronsäurepinakolester, oder $-BF_3^-K^+$ steht,

umgesetzt wird.

Die Verbindungen der Formeln (VI) und (VII) sind bekannt oder lassen sich nach bekannten Verfahren aus den entsprechenden Edukten synthetisieren.

Gegenstand der Erfindung ist weiterhin ein Verfahren zur Herstellung der Verbindungen der Formel (I) und (Ia), wobei Verbindungen der Formel

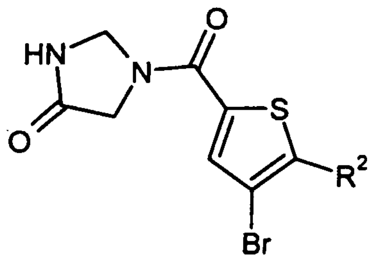

(VIII),

in welcher
$R^2$ die oben angegebene Bedeutung aufweist, unter den oben angegebenen Suzuki-Kupplungsbedingungen mit Verbindungen der Formel

$R^1$-Q (V),

in welcher
$R^1$ die oben angegebene Bedeutung aufweist und

Q für $-B(OH)_2$, einen Boronsäure-Ester, bevorzugt Boronsäurepinakolester, oder $-BF_3^-K^+$ steht,

umgesetzt werden.

Die Verbindungen der Formel (VIII) sind bekannt oder können hergestellt werden, indem Verbindungen der Formel

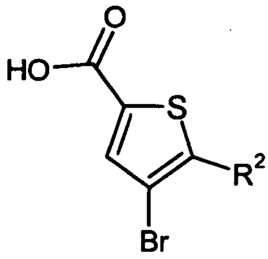

(IX),

in welcher
$R^2$ die oben angegebene Bedeutung aufweist,
mit Imidazolidin-4-on oder einem Salz von Imidazolidin-4-on, entsprechend der oben angegebenen Reaktion von (II) zu (I) bzw. (Ia), umgesetzt werden.

Die Verbindungen der Formel (IX) sind bekannt oder können hergestellt werden, indem in Verbindungen der Formel

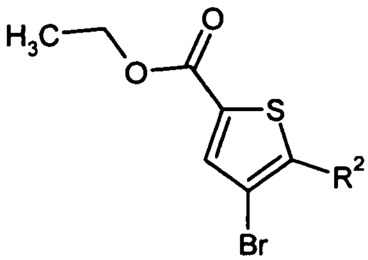

(IV),

in welcher
$R^2$ die oben angegebene Bedeutung aufweist,
der Ester mit einer Base, wie oben bei der Reaktion von (III) zu (II) beschrieben, verseift wird.

Gegenstand der Erfindung ist weiterhin ein Verfahren zur Herstellung der Verbindungen der Formel (I) und (Ia), wobei Verbindungen der Formel

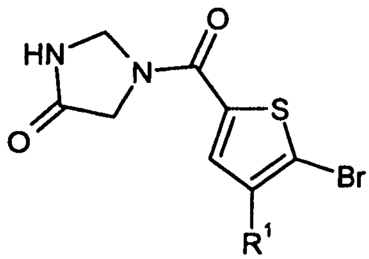

(X),

in welcher
$R^1$ die oben angegebene Bedeutung aufweist,
unter den oben angegebenen Suzuki-Kupplungsbedingungen mit Verbindungen der Formel

$R^2$-Q (VII),

in welcher
$R^2$ die oben angegebene Bedeutung aufweist und

Q für $-B(OH)_2$, einen Boronsäure-Ester, bevorzugt Boronsäurepinakolester, oder $-BF_3^-K^+$ steht,

umgesetzt werden.

Die Verbindungen der Formel (X) sind bekannt oder können hergestellt werden, indem Verbindungen der Formel

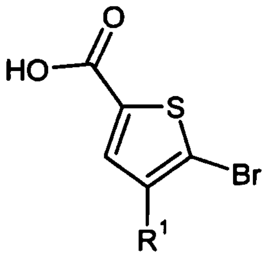

(XI),

in welcher
$R^1$ die oben angegebene Bedeutung aufweist,
mit Imidazolidin-4-on oder einem Salz von Imidazolidin-4-on, entsprechend der oben angegebenen Reaktion von (II) zu (I) bzw. (Ia), umgesetzt werden.

Die Verbindungen der Formel (XI) sind bekannt oder können hergestellt werden, indem in Verbindungen der Formel

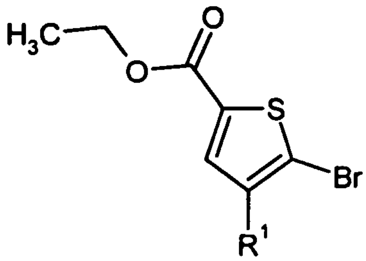

(XII),

in welcher
$R^1$ die oben angegebene Bedeutung aufweist,
der Ester mit einer Base, wie oben bei der Reaktion von (III) zu (II) beschrieben, verseift wird.

Die Verbindungen der Formel (XII) sind bekannt oder können hergestellt werden, indem Verbindungen der Formel

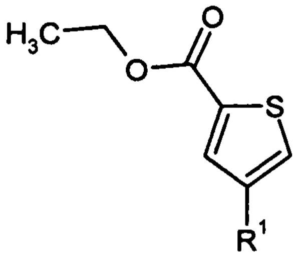

(XIII),

in welcher
$R^1$ die oben angegebene Bedeutung aufweist, bromiert werden.

Inerte Lösungsmittel für die Bromierung sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Kohlenwasserstoffe wie Hexan oder Cyclohexan, organische Carbonsäuren wie Essigsäure oder andere Lösungsmittel wie Ethylacetat, Dimethylformamid oder Dimethylsulfoxid. Ebenso ist es möglich, Gemische der genannten Lösungsmittel einzusetzen. Bevorzugt sind Essigsäure, Diethylether, Dioxan, Tetrahydrofuran, Ethylacetat, Trichlormethan und/oder Tetrachlormethan.

Als Bromierungsmittel eignen sich die üblichen anorganischen oder organischen Reagenzien. Hierzu gehören bevorzugt Brom, *N*-Bromsuccinimid, Kupferdibromid, Pyridinhydrotribromid, Dimethylbenzylammoniumtribromid oder Phenyltrimethylammoniumtribromid. Besonders bevorzugt sind Brom und Kupferdibromid.

Die Bromierung wird im Allgemeinen in einem Temperaturbereich von -20°C bis 150°C, bevorzugt von 0°C bis 80°C durchgeführt. Die Umsetzung kann bei normalem, erhöhtem oder erniedrigtem Druck erfolgen (z.B. von 0.5 bis 5 bar). Im Allgemeinen erfolgt die Bromierung bei Normaldruck.

Die Verbindungen der Formel (XIII) sind bekannt oder können hergestellt werden, indem die Verbindung der

Formel

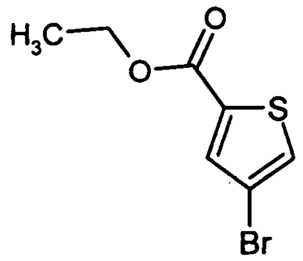

(XIV),

unter den oben angegebenen Suzuki-Kupplungsbedingungen mit Verbindungen der Formel

$R^1$-Q (V),

in welcher

$R^1$ die oben angegebene Bedeutung aufweist und

Q für $-B(OH)_2$, einen Boronsäure-Ester, bevorzugt Boronsäurepinakolester, oder $-BF_3^-K^+$ steht,

umgesetzt wird.

Die Verbindung der Formel (XIV) ist bekannt oder lässt sich nach bekannten Verfahren synthetisieren.

Die Herstellung der erfindungsgemäßen Verbindungen kann durch die folgenden Syntheseschemata verdeutlicht werden.

Syntheseschema I:

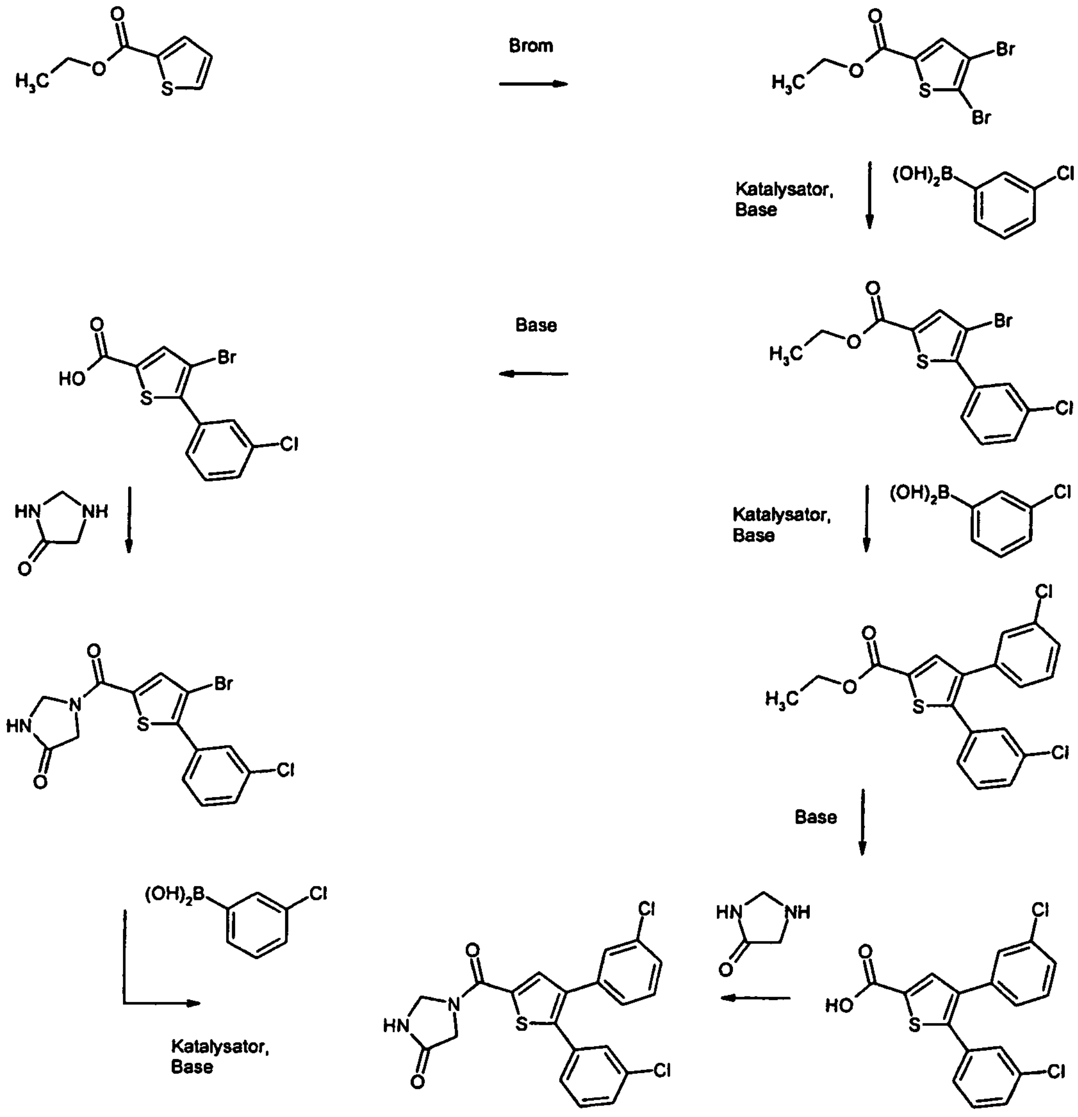

Syntheseschema II:

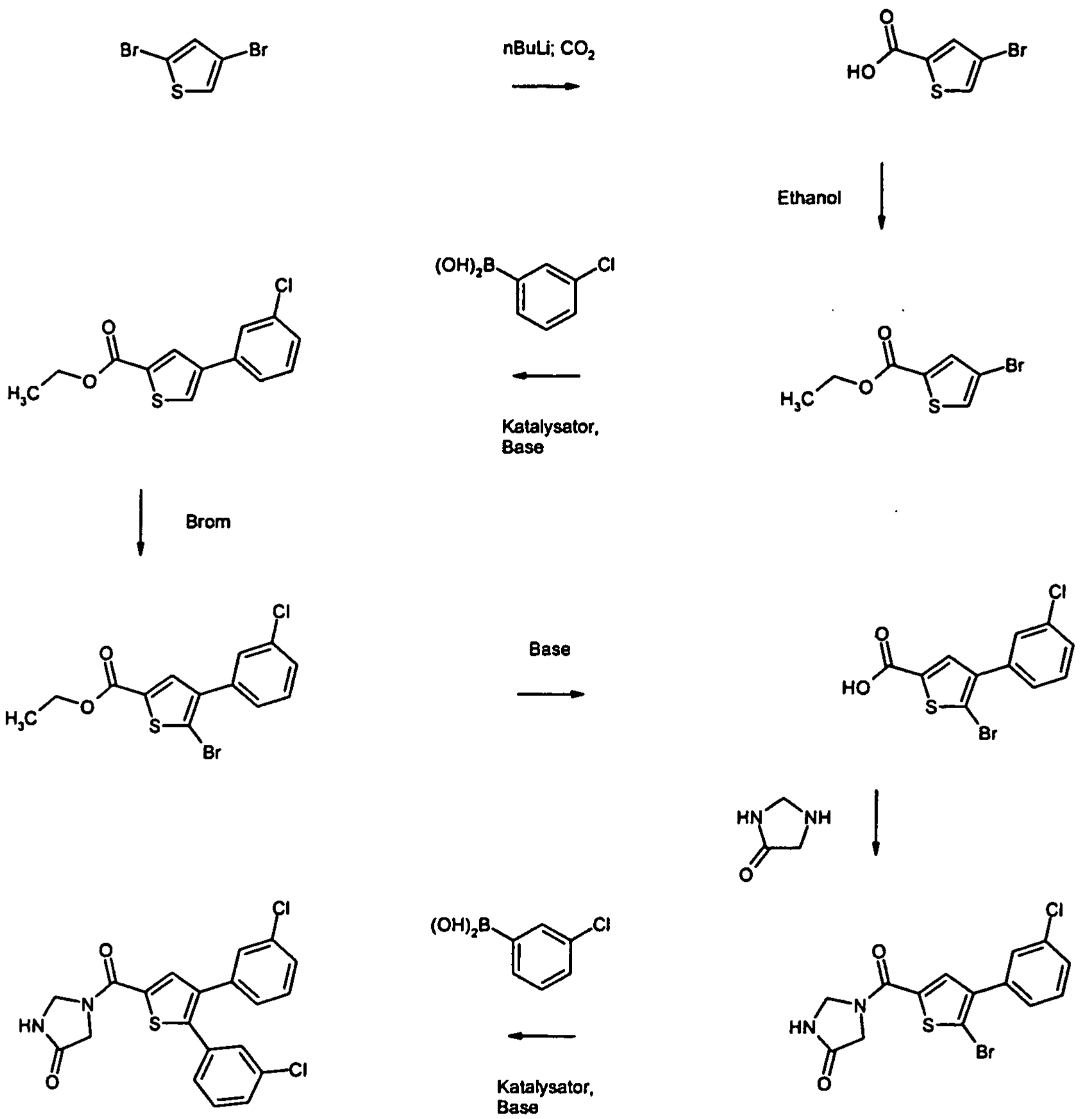


Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Die Verbindungen der vorliegenden Erfindung zeichnen sich insbesondere durch ein vorteilhaftes anti-retrovirales Wirkspektrum aus.

Weiterer Gegenstand der vorliegenden Erfindung ist der Einsatz der erfindungsgemäßen Verbindungen zur Behandlung und/oder Prophylaxe von Erkrankungen, die durch Retroviren hervorgerufen werden, insbesondere von HI-Viren.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer therapeutisch wirksamen Menge der erfindungsgemäßen Verbindungen.

Als Indikationsgebiete in der Humanmedizin können beispielsweise genannt werden:

1.) Die Behandlung und Prophylaxe von menschlichen Retrovirusinfektionen

2.) Die Behandlung und Prophylaxe von durch HIV-1 (Virus der humanen Immundefizienz; früher HTLV III/LAV genannt) und HIV-2 verursachten Infektionen und Erkrankungen (AIDS) und den damit assoziierten Stadien wie ARC (AIDS related complex) und LAS (Lymphadenophathie-Syndrom) sowie der durch dieses Virus verursachten Immunschwäche und Enzephalopathie.

3.) Die Behandlung von HIV-Infektionen hervorgerufen durch einfach-, mehrfach- oder multi-resistente HI-Viren.

Resistente HI-Viren bedeutet z.B. Viren mit Resistenzen gegen nukleosidische RT-Inhibitoren (NRTI), nicht-nukleosidische RT-Inhibitoren (NNRTI) oder Proteaseinhibitoren (PI) oder Viren mit Resistenzen gegen andere Wirkprinzipien, z.B. T20 (Fusionsinhibitoren).

4.) Für die Behandlung oder die Prophylaxe des AIDS-carrier Zustandes (AIDS-Überträger-Zustand).

5.) Für die Behandlung oder die Prophylaxe einer HTLV-I oder HTLV-II Infektion

Als Indikationen in der Tiermedizin können beispielsweise angeführt werden:

Infektionen mit

a) Maedivisna (bei Schafen und Ziegen)

b) progressivem Pneumonievirus (PPV) (bei Schafen und Ziegen)

c) caprine arthritis encephalitis Virus (bei Schafen und Ziegen)

d) Zwoegerziekte Virus (bei Schafen)

e) infektiösem Virus der Anämie (des Pferdes)

f) Infektionen verursacht durch das Katzenleukämievirus

g) Infektionen verursacht durch das Virus der Katzen-Immundefizienz (FIV)

h) Infektionen verursacht durch das Virus der Affen-Immundefizienz (SIV)

Bevorzugt werden aus dem Indikationsgebiet in der Humanmedizin die oben aufgeführten Punkte 2, 3 und 4.

Insbesondere geeignet sind die Substanzen zur Bekämpfung von HI-Viren, die Resistenzen gegen bekannte nicht-nukleosidische Inhibitoren der Reversen Transkripatse, wie z.B. Efavirenz oder Nevirapin, zeigen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Die erfindungsgemäßen Verbindungen können auch vorteilhaft, insbesondere in den oben aufgeführten Punkten 2, 3 und 4, als Bestandteile einer Kombinationstherapie mit einer oder mehreren anderen, in diesen Anwendungsbereichen aktiven Verbindungen eingesetzt werden. Beispielhaft können diese Verbindungen in Kombination mit wirksamen Dosen von antiviral wirksamen Substanzen, die auf den unten aufgeführten Wirkprinzipien beruhen, eingesetzt werden:

Inhibitoren der HIV Protease; beispielhaft seien genannt: Saquinavir, Indinavir, Ritonavir, Nelfinavir, Amprenavir, Lopinavir, Atazanavir, Fosamprenavir, Tipranavir, Darunavir;

Nukleosidische, nukleotidische und nicht-nukleosidische Inhibitoren der HIV Reversen Transkriptase; beispielhaft seien genannt: Zidovudin, Lamivudin, Didanosin, Zalcitabin, Stavudin, Lamivudin, Abacavir, Tenofovir, Adefovir, Emtricitabin, Amdoxovir, Apricitabin, Racivir, Nevirapin, Delavirdin, Efavirenz, Etravirin, Rilpivirin, UK-453,061;

Inhibitoren der HIV Integrase, beispielhaft seien genannt: Raltegravir, Elvitegravir;

Inhibitoren der HIV Fusion; beispielhaft sei genannt: Enfuvirtid;

Inhibition der CXCR4/CCR5/gp120 Wechselwirkung; beispielhaft seien genannt: Maraviroc, Vicriviroc, INCB009471, AMD-070;

Inhibition der Polyproteinreifung; beispielhaft sei genannt: Bevirimat.

Diese Auswahl soll zur Verdeutlichung der Kombinationsmöglichkeiten, nicht jedoch zur Einschränkung auf die hier aufgeführten Beispiele dienen; prinzipiell ist jede Kombination der erfindungsgemäßen Verbindungen mit antiviral wirksamen Substanzen als im Rahmen der Erfindung zu betrachten.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich sowohl in der Human- als auch in der Veterinärmedizin als vorteilhaft erwiesen, den oder die erfindungsgemäßen Wirkstoffe in Gesamtmengen von 0.1 bis 200 mg/kg, vorzugsweise 1 bis 100 mg/kg Körpergewicht je 24 Stunden, gegebenenfalls in Form mehrerer Einzelgaben, zur Erzielung des gewünschten Ergebnisses zu verabreichen. Eine Einzelgabe enthält den oder die Wirkstoffe vorzugsweise in Mengen von 1 bis 80 mg/kg, insbesondere 1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

**A) Beispiele**

**Abkürzungen:**

| | |
|---|---|
| aq. | wässrig, wässrige Lösung |
| DCI | direkte chemische Ionisation (bei MS) |
| DMA | *N,N*-Dimethylacetamid |
| DMF | *N,N*-Dimethylformamid |
| DMSO | Dimethylsulfoxid |
| d. Th. | der Theorie (bei Ausbeute) |
| EDC | N'-(3-Dimethylaminopropyl)-*N*-ethylcarbodümid x HCl |
| eq. | Äquivalent(e) |
| ESI | Elektrospray-Ionisation (bei MS) |
| h | Stunde(n) |
| HATU | O-(7-Azabenzotriazol-1-yl)-*N,N,N',N'*-tetramethyluronium-hexafluorophosphat |
| HPLC | Hochdruck-, Hochleistungsflüssigchromatographie |
| konz. | konzentriert |
| LC-MS | Flüssigchromatographie-gekoppelte Massenspektroskopie |
| min | Minute(n) |
| MS | Massenspektroskopie |
| NMR | Kernresonanzspektroskopie |
| PyBOP | Benzotriazol-1-yloxytris(pyrrolidino)phosphonium-hexafluorophosphat |
| $R_t$ | Retentionszeit (bei HPLC) |
| RT | Raumtemperatur |
| TBTU | O-(Benzotriazol-1-yl)-*N,N,N',N'*-tetramethyluronium-tetrafluoroborat |
| TFA | Trifluoressigsäure |
| THF | Tetrahydrofuran |
| TMOF | Trimethylorthoformiat |

**LC-MS/GC-MS-Methoden:**

Methode 1:

Gerätetyp MS: Micromass ZQ; Gerätetyp HPLC: HP 1100 Series; UV DAD; Säule: Phenomenex Gemini 3p 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

Methode 2:

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

Methode 3:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

Methode 4:

Instrument: Micromass Quattro LCZ mit HPLC Agilent Serie 1100; Säule: Phenomenex Onyx Monolithic C18, 100 mm x 3 mm. Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A→ 6 min 5%A; Fluss: 2 ml/min; Ofen: 40°C; UV-Detektion: 208-400 nm.

Methode 5:

Instrument: Micromass Quattro Premier mit Waters UPLC Acquity; Säule: Thermo Hypersil GOLD 1,9 μ 50 mm x 1 mm; Eluent A: 1 1 Wasser + 0,5 ml 50%ige Ameisensäure, Eluent B: 1 1 Acetonitril + 0,5 ml 50%ige Ameisensäure; Gradient: 0.0 min 90%A → 0.1 min 90%A → 1.5 min 10%A → 2.2 min 10%A Ofen: 50°C; Fluss: 0.33 ml/min; UV-Detektion: 210 nm.

Methode 6:

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

Methode 7:

Gerätetyp MS: Micromass ZQ; Gerätetyp HPLC: Waters Alliance 2795; Säule: Phenomenex Synergi 2.5 μ MAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.01 min 90%A; Fluss: 2 ml/min; Ofen: 50°C; UV-Detektion: 210 nm.

Methode 8:

Instrument: Micromass Quattro LCZ mit HPLC Agilent Serie 1100; Säule: Phenomenex Gemini 3μ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208-400 nm.

Methode 9:

Instrument: Micromass Quattro LCZ mit HPLC Agilent Serie 1100; Säule: Phenomenex Synergi 2.5 μ MAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.1 min 90%A; Fluss: 2 ml/min; Ofen: 50°C; UV-Detektion: 208- 400 nm.

Methode 10:

Gerätetyp MS: Waters (Micromass) Quattro Micro; Gerätetyp HPLC: Agilent Serie 1100; Säule: Thermo Hypersil GOLD 3μ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 3.0 min 10%A → 4.0 min 10%A → 4.01 min 100%A (Flow 2.5ml) → 5.00 min 100%A; Ofen: 50°C; Fluss: 2 ml/min; UV-Detektion: 210 nm.

Methode 11:

Instrument: Micromass GCT, GC6890; Säule: Restek RTX-35, 15 m x 200 μm x 0.33 μm; konstanter Fluss mit Helium: 0.88 ml/min; Ofen: 70°C; Inlet: 250°C; Gradient: 70°C, 30°C/min → 310°C (3 min halten).

Methode 12:

Instrument: Micromass GCT, GC6890; Säule: Restek RTX-35, 15 m x 200 μm x 0.33 μm; konstanter Fluss mit Helium: 0.88 ml/min; Ofen: 70°C; Inlet: 250°C; Gradient: 70°C, 30°C/min → 310°C (12 min halten).

**Ausgangsverbindungen und Intermediate:**

**Beispiel 1A**

$N^2$-Benzylglycinamid

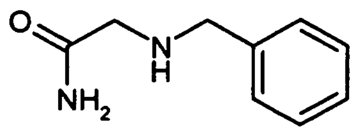

44.2 g (0.40 mol) Glycinamid-hydrochlorid werden in 2.2 1 Dichlormethan bei Raumtemperatur unter Argon vorgelegt, mit 112 ml (0.80 mol) Triethylamin versetzt und über Nacht bei Raumtemperatur gerührt. Dann werden 42.5 g (0.40 mol) Benzaldehyd zugegeben und die Reaktionsmischung wird über Nacht am Wasserabscheider unter Rückfluss erhitzt. Man engt ein, löst den Rückstand in 400 ml Tetrahydrofuran/Methanol (1:1), versetzt bei 0°C portionsweise mit 16.7 g (0.44 mol) Natriumborhydrid und rührt zwei Tage bei Raumtemperatur. Die Suspension wird abgesaugt, das Filtrat eingeengt und im Hochvakuum getrocknet. Der Rückstand wird in Essigsäureethylester gerührt, der Niederschlag abfiltriert, das Filtrat eingeengt und der Rückstand in Toluol über Nacht gerührt. Nach Filtration des Feststoffs und anschließendem Trocknen im Hochvakuum erhält man 56.5 g (84% d. Th.) der Titelverbindung.
$^1$H-NMR (400 MHz, DMSO-$d_6$): $\delta$ = 7.36-7.28 (m, 4H), 7.27-7.19 (m, 1H), 3.66 (d, 2H), 3.02 (d, 2H).
LC-MS (Methode 10): $R_t$ = 0.40 min; MS (ESIpos): m/z = 165 $[M+H]^+$.

**Beispiel 2A**

1-Benzyl-3-(hydroxymethyl)imidazolidin-4-on

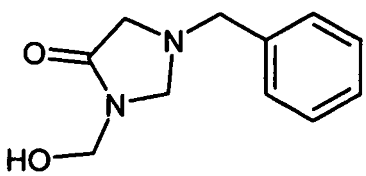

56.5 g (0.34 mol) der Verbindung aus Beispiel 1A werden mit 172 ml (6.20 mol) 37%iger Formaldehyd-Lösung versetzt und 30 Minuten unter Rückfluss erhitzt. Es wird mit Dichlormethan extrahiert, und die vereinigten organischen Phasen werden über Natriumsulfat getrocknet, filtriert und eingeengt. Man erhält 74.5 g (100% d. Th.) der Titelverbindung.
LC-MS (Methode 5): $R_t$ = 0.51 min; MS (ESIpos): m/z = 207 $[M+H]^+$.

**Beispiel 3A**

1-Benzylimidazolidin-4-on-trifluoracetat

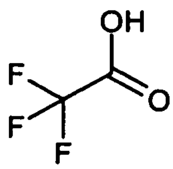

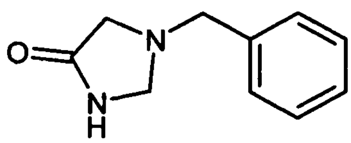

74.5 g (0.36 mol) der Verbindung aus Beispiel 2A werden für 6 h bei 150°C im Hochvakuum unter Abdestillieren flüchtiger Reaktionsprodukte erhitzt. Die Aufreinigung erfolgt durch HPLC (Säule: Sunfire C18 5µ, 250 x 20 mm; Eluent:

0.2% Trifluoressigsäure/Wasser-Acetonitril-Gradient). Man erhält 28.4 g (27% d. Th.) der Titelverbindung.
$^{1}$H-NMR (400 MHz, DMSO-$d_6$): $\delta$ = 8.75 (s, 1H), 7.48-7.39 (m, 5H), 4.41 (s, 2H), 4.22 (s, 2H), 3.54 (s, 2H).
LC-MS (Methode 10): $R_t$ = 0.94 min; MS (ESIpos): m/z = 177 [M-$CF_3COOH$+H]$^+$.

**Beispiel 4A**

Imidazolidin-4-on-trifluoracetat

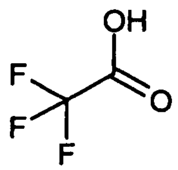

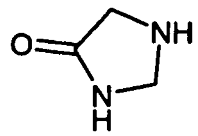

28.4 g (97.9 mmol) der Verbindung aus Beispiel 3A werden in 750 ml Ethanol gelöst und unter Argon mit 4.5 g (42.3 mmol) Palladium auf Aktivkohle (5%ig) versetzt. Es wird 24 h unter Wasserstoffatmosphäre hydriert. Die Suspension wird über Celite filtriert, eingeengt und am Hochvakuum getrocknet. Man erhält 19.2 g (98% d. Th.) der Titelverbindung.
$^{1}$H-NMR (400 MHz, DMSO-$d_6$): $\delta$ = 10.1 (s, 2H), 8.89 (s, 1H), 4.55 (s, 2H), 3.63 (s, 2H).
GC-MS (Methode 11): $R_t$ = 3.92 min; MS (EIpos): m/z = 86 [M-$CF_3COOH$]$^+$.

**Beispiel 5A**

3-Fluor-5-(4,4,5,5-tetramethyl-1,3,2-dioxaborolan-2-yl)benzolcarbonitril

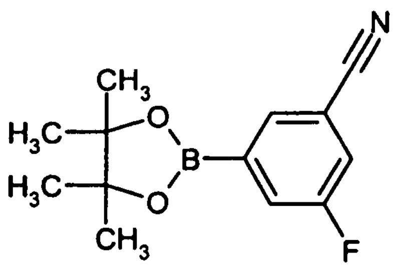

3.60 g (18.0 mmol) 3-Brom-5-fluorbenzolcarbonitril, 5.03 g (19.8 mmol) 4,4,4',4',5,5,5',5'-Octamethyl-2,2'bi-1,3,2-dioxaborolan und 5.30 g (54.0 mmol) Kaliumacetat werden unter Argon in 72 ml entgastem 1,4-Dioxan/DMSO (10/1) vorgelegt und mit 441 mg (0.54 mmol) 1,1'-Bis(diphenylphosphin)ferrocendichlorpalladium(II)-Dichlormethan-Komplex versetzt. Es wird über Nacht bei 90°C gerührt. Anschließend gibt man Wasser hinzu, trennt die Phasen, extrahiert die wässrige Phase mit Essigsäureethylester und engt die vereinigten organischen Phasen ein. Das Rohprodukt wird mittels Flash-Chromatographie (Laufmittel: Cyclohexan/Essigsäureethylester 10:1) gereinigt. Man erhält 4.48 g (92% d. Th.) der Titelverbindung.
$^{1}$H-NMR (400 MHz, DMSO-$d_6$): $\delta$ = 8.01 (ddd, 1H), 7.82 (s, 1H), 7.70 (ddd, 1H), 1.32 (s, 12H).
GC-MS (Methode 11): $R_t$ = 4.94 min; MS (EIpos): m/z = 247 [M]$^+$.

**Beispiel 6A**

4-Bromthiophen-2-carbonsäure

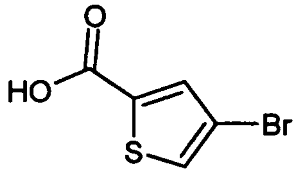

1.00 g (4.13 mmol) 2,4-Dibromthiophen werden unter Argon in 15 ml Diethylether vorgelegt und auf -78°C gekühlt. Es werden 2.6 ml (4.13 mmol) einer 1.6N n-Butyllithium-Lösung in Hexan zugetropft und das Reaktionsgemisch wird 5 Minuten gerührt. Das Reaktionsgemisch wird auf eine Mischung aus Trockeneis und 15 ml Diethylether gegeben und 10 Minuten gerührt. Anschließend wird mit Wasser verdünnt und die Phasen werden getrennt. Die wässrige Phase wird angesäuert und mit Essigsäureethylester extrahiert, die organische Phase wird über Natriumsulfat getrocknet, filtriert und eingeengt. Man erhält 0.66 g (74% d. Th.) der Titelverbindung.
$^1$H-NMR (400 MHz, DMSO-$d_6$): $\delta$ = 13.5 (s, 1H), 8.02 (d, 1H), 7.69 (d, 1H).
LC-MS (Methode 1): $R_t$ = 1.88 min; MS (ESIpos): m/z = 207 $[M+H]^+$.

**<u>Beispiel 7A</u>**

4-Bromthiophen-2-carbonsäureethylester

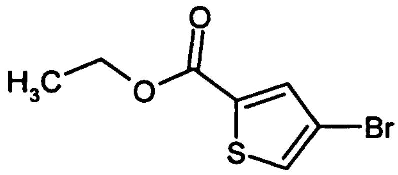

663 mg (3.20 mmol) der Verbindung aus Beispiel 6A werden in 25 ml Ethanol vorgelegt, mit 0.1 ml konzentrierter Schwefelsäure versetzt und über Nacht unter Rückfluss erhitzt. Nach dem Abkühlen auf Raumtemperatur wird gesättigte wässrige Natriumhydrogencarbonat-Lösung zugegeben, mit Wasser verdünnt und mit Essigsäureethylester extrahiert. Die organische Phase wird über Natriumsulfat getrocknet, filtriert und eingeengt. Man erhält 700 mg (89% d. Th.) der Titelverbindung.
$^1$H-NMR (400 MHz, DMSO-$d_6$): $\delta$ = 8.07 (d, 1H), 7.78 (d, 1H), 4.30 (q, 2H), 1.30 (t, 3H).
GC-MS (Methode:11): $R_t$ = 4.69 min; MS (EIpos): m/z = 234 $[M]^+$.

**<u>Beispiel 8A</u>**

4,5-Dibromthiophen-2-carbonsäureethylester

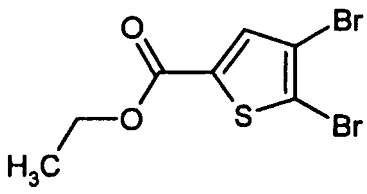

16.5 ml (320 mmol) Brom werden in 120 ml Essigsäure vorgelegt und 10.0 g (64.0 mmol) Thiophen-2-carbonsäureethylester in 40 ml Essigsäure gelöst zugetropft. Es wird über Nacht bei 60°C gerührt. Die Reaktionsmischung wird eingeengt, der Rückstand mit gesättigter wässriger Natriumhydrogencarbonat-Lösung versetzt und mit Dichlormethan extrahiert. Man trocknet die organische Phase über Magnesiumsulfat, filtriert und engt ein. Das Rohprodukt wird aus Diethylether umkristallisiert. Man erhält 7.80 g (39% d. Th.) der Titelverbindung.
$^1$H-NMR (400 MHz, DMSO-$d_6$): $\delta$ = 7.79 (s, 1H), 4.30 (q, 2H), 1.29 (t, 3H).
GC-MS (Methode 11): $R_t$ = 5.55 min; MS (EIpos): m/z = 314 $[M]^+$.

**Beispiel 9A**

4-Brom-5-(3-chlor-5-fluorphenyl)thiophen-2-carbonsäureethylester

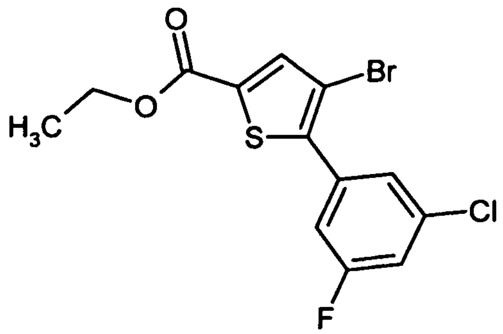

3.71 g (11.8 mmol) der Verbindung aus Beispiel 8A und 2.16 g (12.4 mmol) 3-Chlor-5-fluorphenylboronsäure werden unter Argon in 125 ml entgastem Toluol/Wasser (2.2/1) vorgelegt und mit 6.75 g (63.7 mmol) Natriumcarbonat und 409 mg (0.35 mmol) Tetrakis(triphenylphosphin)palladium(0) versetzt. Es wird über Nacht bei 60°C gerührt. Die Reaktionsmischung wird mit Wasser versetzt, die Phasen werden getrennt, die wässrige Phase wird mit Dichlormethan extrahiert, und die vereinigten organischen Phasen werden über Natriumsulfat getrocknet, filtriert und eingeengt. Das Rohprodukt wird über präparative HPLC (RP18-Säule; Eluent: Acetonitril/Wasser-Gradient) gereinigt. Man erhält 1.17 g (27% d. Th.) der Titelverbindung.
$^1$H-NMR (400 MHz, DMSO-$d_6$): $\delta$ = 7.90 (s, 1H), 7.66-7.54 (m, 3H), 4.34 (q, 2H), 1.31 (t, 3H).
LC-MS (Methode 1): $R_t$ = 3.22 min; MS (ESIpos): m/z = 363 $[M+H]^+$.

**Beispiel 10A**

4-Brom-5-(3-chlorphenyl)thiophen-2-carbonsäureethylester

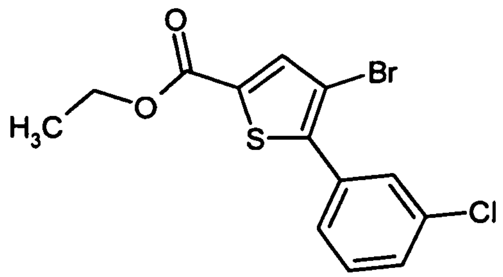

Die Herstellung der Titelverbindung erfolgt ausgehend von der Verbindung aus Beispiel 8A analog zur Synthese der Verbindung aus Beispiel 9A. Man erhält 125 mg (57% d. Th.) der Titelverbindung.
$^1$H-NMR (400 MHz, DMSO-$d_6$): $\delta$ = 7.88 (s, 1H), 7.77-7.73 (m, 1H), 7.67-7.54 (m, 3H), 4.33 (q, 2H), 1.31 (t, 3H).
GC-MS (Methode 11): $R_t$ = 7.91 min; MS (EIpos): m/z = 346 $[M]^+$.

**Beispiel 11A**

5-(3-Chlor-5-fluorphenyl)-4-(3-cyanphenyl)thiophen-2-carbonsäureethylester

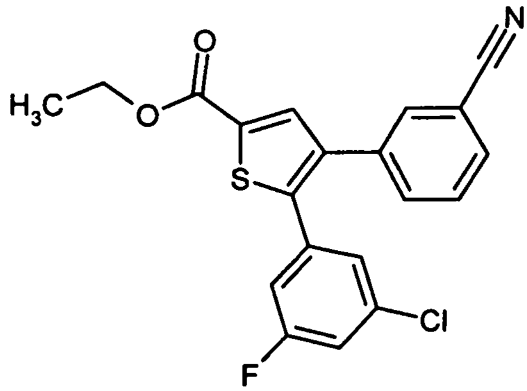

400 mg (1.10 mmol) der Verbindung aus Beispiel 9A und 242 mg (1.65 mmol) 3-Cyanphenylboronsäure werden unter Argon in 15.7 ml 1,2-Dimethoxyethan vorgelegt und mit 5.8 ml (5.50 mmol) wässriger Natriumhydrogencarbonat-Lösung (10%ig) und 38.1 mg (0.03 mmol) Tetrakis(triphenylphosphin)palladium(0) versetzt. Es wird über Nacht bei 80°C gerührt. Die Reaktionsmischung wird über präparative HPLC (RP18-Säule; Eluent: Acetonitril/Wasser-Gradient unter Zusatz von 0.1% Ameisensäure) gereinigt. Man erhält 130 mg (27% d. Th.) der Titelverbindung.
GC-MS (Methode 12): $R_t$ = 9.85 min; MS (EIpos): m/z = 385 $[M]^+$.

**Beispiel 12A**

4-(3-Chlor-4-fluorphenyl)-5-(3-chlor-5-fluorphenyl)thiophen-2-carbonsäureethylester

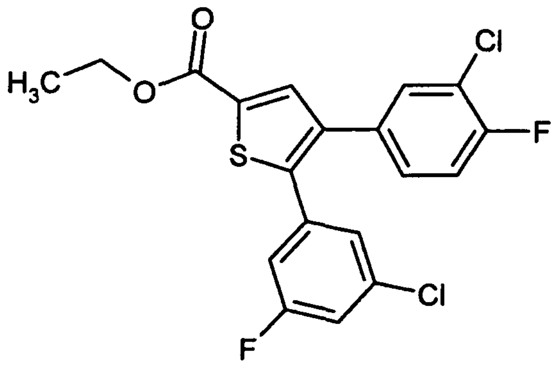

Die Herstellung der Titelverbindung erfolgt ausgehend von der Verbindung aus Beispiel 9A analog zur Synthese der Verbindung aus Beispiel 11A. Man erhält 59.8 mg (46% d. Th.) der Titelverbindung.
$^1$H-NMR (400 MHz, DMSO-$d_6$): $\delta$ = 7.96 (s, 1H), 7.61 (dd, 1H), 7.52 (dt, 1H), 7.41 (t, 1H), 7.26-7.16 (m, 3H), 4.34 (q, 2H), 1.32 (t, 3H).
LC-MS (Methode 1): $R_t$ = 3.36 min; MS (ESIpos): m/z = 317 $[M+H]^+$.

**Beispiel 13A**

4-(3-Chlorphenyl)thiophen-2-carbonsäureethylester

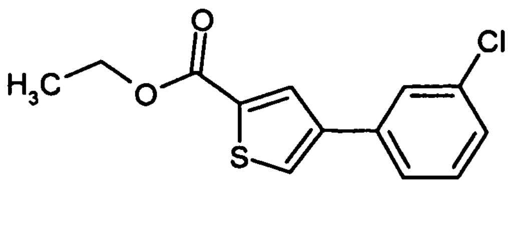

5.00 g (21.3 mmol) der Verbindung aus Beispiel 7A und 4.99 g (31.9 mmol) 3-Chlorphenylboronsäure werden unter Argon in 150 ml 1,2-Dimethoxyethan vorgelegt und mit 113 ml (106 mmol) wässriger Natriumhydrogencarbonat-Lösung (10%ig) und 737 mg (0.64 mmol) Tetrakis(triphenylphosphin)palladium(0) versetzt. Es wird 1.5 Stunden bei 80°C gerührt. Anschließend gibt man 1N wässrige HCl-Lösung zu, extrahiert mit Essigsäureethylester, engt ein und reinigt mittels Flash-Chromatographie (Laufmittel: Cyclohexan/Essigsäureethylester-Gradient). Man erhält 5.60 g (88% d. Th.) der Titelverbindung.
$^1$H-NMR (400 MHz, DMSO-$d_6$): $\delta$ = 8.38 (d, 1H), 8.27 (d, 1H), 7.90 (t, 1H), 7.77 (dt, 1H), 7.46 (t, 1H), 7.41-7.37 (m, 1H), 4.33 (q, 2H), 1.32 (t, 3H).
LC-MS (Methode 5): $R_t$ = 1.47 min; MS (ESIpos): m/z = 267 $[M+H]^+$.

**Beispiel 14A**

5-Brom-4-(3-chlorphenyl)thiophen-2-carbonsäureethylester

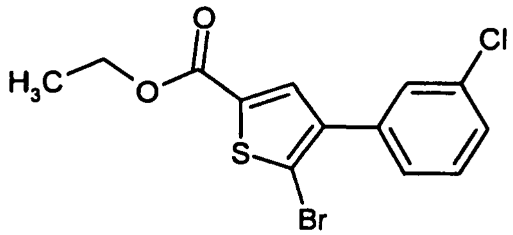

0.01 ml (0.22 mmol) Brom wird in 1 ml Essigsäure vorgelegt und 58.0 mg (0.22 mmol) der Verbindung aus Beispiel 13A in 1 ml Essigsäure gelöst, werden zugetropft. Es wird über Nacht bei 60°C gerührt. Anschließend wird 0.01 ml (0.22 mmol) Brom zugegeben und weitere 24 Stunden bei 60°C gerührt. Die Reaktionsmischung wird mit Wasser versetzt, der entstehende Niederschlag abgesaugt, mit Wasser gewaschen und am Hochvakuum getrocknet. Man erhält 43.0 mg (54% d. Th.) der Titelverbindung.
$^1$H-NMR (400 MHz, DMSO-$d_6$): $\delta$ = 7.88 (s, 1H), 7.68-7.66 (m, 1H), 7.59-7.54 (m, 1H), 7.54-7.51 (m, 2H), 4.32 (q, 2H), 1.30 (t, 3H).
LC-MS (Methode 1): $R_t$ = 3.35 min; MS (ESIpos): m/z = 345 $[M+H]^+$.

**Beispiel 15A**

5-Brom-4-(3-chlorphenyl)thiophen-2-carbonsäure

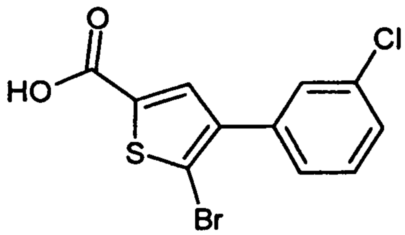

41.0 mg (0.12 mmol) der Verbindung aus Beispiel 14A werden in 3 ml Tetrahydrofuran vorgelegt und bei Raumtemperatur mit 28.4 mg (1.19 mmol) Lithiumhydroxid und 0.8 ml Wasser versetzt. Man rührt bei Raumtemperatur über Nacht, gibt anschließend 1N wässrige HCl-Lösung bis zu einem sauren pH-Wert hinzu, extrahiert dreimal mit Dichlormethan, trocknet über Natriumsulfat, filtriert und engt ein. Man erhält 35.0 mg (83% d. Th.) der Titelverbindung.
$^1$H-NMR (400 MHz, DMSO-$d_6$): $\delta$ = 13.6 (s, 1H), 7.77 (s, 1H), 7.66 (s, 1H), 7.58-7.54 (m, 1H), 7.53-7.50 (m, 2H).
LC-MS (Methode 1): $R_t$ = 2.85 min; MS (ESIpos): m/z = 317 $[M+H]^+$.

**Beispiel 16A**

1-{[5-Brom-4-(3-chlorphenyl)thiophen-2-yl]carbonyl}imidazolidin-4-on

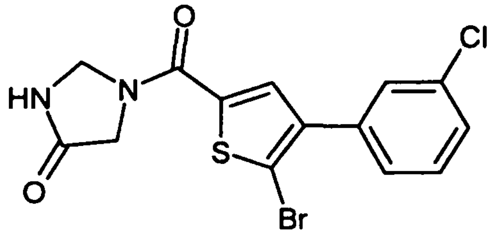

35.0 mg (0.11 mmol) der Verbindung aus Beispiel 15A, 10.4 mg (0.12 mmol) 4-Imidazolidinon und 86.0 mg (0.17 mmol) PyBOP werden in 2.8 ml Tetrahydrofuran vorgelegt und bei Raumtemperatur mit 0.04 ml (0.23 mmol) N,N-Diisopropylethylamin versetzt. Man rührt bei Raumtemperatur über Nacht und reinigt anschließend die Reaktionsmischung über präparative HPLC (RP18-Säule; Eluent: Acetonitril/Wasser-Gradient). Man erhält 27.0 mg (62% d. Th.) der Titelverbindung.
$^1$H-NMR (400 MHz, DMSO-$d_6$): $\delta$ = 8.77 (s, 1H), 7.77 (s, 1H), 7.72 (s, 1H), 7.64-7.59 (m, 1H), 7.55-7.50 (m, 2H), 5.27 (s, 0.5H), 4.85 (s, 1.5H), 4.44 (s, 1.5H), 3.95 (s, 0.5H).
LC-MS (Methode 7): $R_t$ = 1.80 min; MS (ESIpos): m/z = 385 $[M+H]^+$.

**Beispiel 17A**

5-(3-Chlorphenyl)-4-(3-cyanphenyl)thiophen-2-carbonsäureethylester

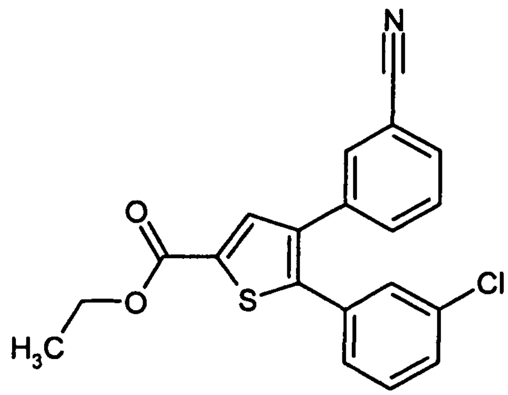

3.20 g (9.26 mmol) der Verbindung aus Beispiel 10A werden unter Argon in 100 ml 1,2-Dimethoxyethan vorgelegt und mit 1.36 g (9.26 mmol) 3-Cyanphenylboronsäure, 9.05 g (27.8 mmol) Cäsiumcarbonat, 309 mg (0.65 mmol) Dicyclohexyl[2',4',6'-tri(propan-2-yl)biphenyl-2-yl]phosphan und 62.4 mg (0.28 mmol) Palladium(II)acetat versetzt. Es wird drei Stunden bei 50°C gerührt. Anschließend wird die Reaktionsmischung mit Wasser verdünnt, mit Dichlormethan extrahiert, die organische Phase über Magnesiumsulfat getrocknet, filtriert und eingeengt. Das Rohprodukt wird durch Flash-Chromatographie (Laufmittel: Cyclohexan/Essigsäureethylester-Gradient) an Kieselgel gereinigt. Man erhält 1.71 g (50% d. Th.) der Titelverbindung.
$^1$H-NMR (400 MHz, DMSO-$d_6$): $\delta$ = 8.00 (s, 1H), 7.87-7.84 (m, 1H), 7.83-7.78 (m, 1H), 7.57-7.51 (m, 2H), 7.51-7.47 (m, 1H), 7.42 (t, 1H), 7.38 (t, 1H), 7.25 (dt, 1H), 4.35 (q, 2H), 1.32 (t, 3H).
LC-MS (Methode 1): $R_t$ = 3.23 min; MS (ESIpos): m/z = 368 $[M+H]^+$.

**Beispiel 18A**

5-(3-Chlor-5-fluorphenyl)-4-(3-cyan-4-fluorphenyl)thiophen-2-carbonsäureethylester

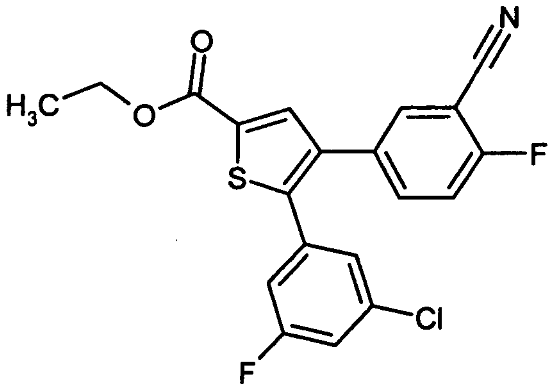

220 mg (0.61 mmol) der Verbindung aus Beispiel 9A werden unter Argon in 6.2 ml 1,2-Dimethoxyethan vorgelegt und mit 100 mg (0.61 mmol) 3-Cyan-4-fluorphenyl-boronsäure, 593 mg (1.82 mmol) Cäsiumcarbonat, 20.0 mg (0.04 mmol) Dicyclohexyl[2',4',6'-tri(propan-2-yl)biphenyl-2-yl]phosphan und 4.0 mg (0.02 mmol) Palladium(II)acetat versetzt. Es wird über Nacht bei 50°C gerührt. Es wird erneut die gleiche Menge Katalysator und Ligand zugegeben und über Nacht bei 50°C gerührt. Die Reaktionsmischung wird über präparative HPLC (RP18-Säule; Eluent: Acetonitril/Wasser-Gradient unter Zusatz von 0.1% Ameisensäure) gereinigt. Man erhält 86.0 mg (35% d. Th.) der Titelverbindung.
$^1$H-NMR (400 MHz, DMSO-$d_6$): $\delta$ = 8.04-8.00 (m, 2H), 7.61-7.47 (m, 3H), 7.27-7.24 (m, 1H), 7.19 (dt, 1H), 4.35 (q, 2H), 1.32 (t, 3H).
LC-MS (Methode 1): $R_t$ = 3.12 min; MS (ESIpos): m/z = 404 $[M+H]^+$.

**Beispiel 19A**

5-(3-Chlor-5-fluorphenyl)-4-(3-cyanphenyl)thiophen-2-carbonsäure

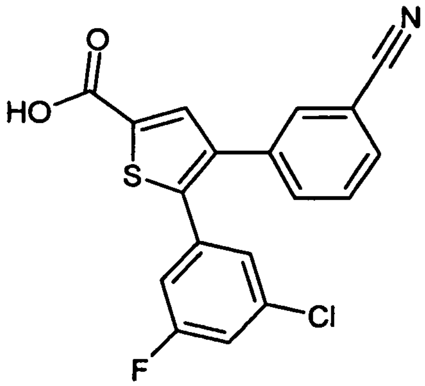

130 mg (0.34 mmol) der Verbindung aus Beispiel 11A werden in 6.1 ml Tetrahydrofuran vorgelegt und bei Raumtemperatur mit 80.7 mg (3.37 mmol) Lithiumhydroxid und 2.0 ml Wasser versetzt. Man rührt bei Raumtemperatur über Nacht, gibt anschließend 1N wässrige HCl-Lösung bis zu einem sauren pH-Wert hinzu, extrahiert mit Essigsäureethylester, trocknet über Natriumsulfat, filtriert und engt ein. Man erhält 119 mg (89% d. Th.) der Titelverbindung.
$^1$H-NMR (400 MHz, DMSO-$d_6$): $\delta$ = 13.5 (s, 1H), 7.93 (s, 1H), 7.87-7.84 (m, 1H), 7.84-7.80 (m, 1H), 7.57-7.49 (m, 3H), 7.23-7.20 (m, 1H), 7.16 (ddd, 1H).
LC-MS (Methode 1): $R_t$ = 2.68 min; MS (ESIpos): m/z = 358 $[M+H]^+$.

**Beispiel 20A**

4-(3-Chlor-4-fluorphenyl)-5-(3-chlor-5-fluorphenyl)thiophen-2-carbonsäure

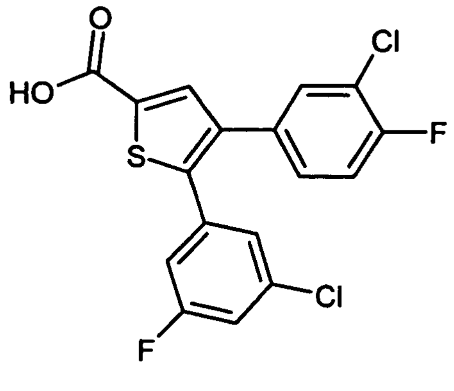

Die Herstellung der Titelverbindung erfolgt ausgehend von der Verbindung aus Beispiel 12A analog zur Synthese der Verbindung aus Beispiel 19A. Die organische Phase wird eingeengt und durch Flash-Chromatographie (Laufmittel: Cyclohexan/Essigsäureethylester/Ethylacetat:Methanol 1:1-Gradient) über Kieselgel gereinigt. Man erhält 0.43 g (100% d. Th.) der Titelverbindung.
$^1$H-NMR (400 MHz, DMSO-$d_6$): $\delta$ = 13.5 (s, 1H), 7.87 (s, 1H), 7.59 (dd, 1H), 7.51 (dt, 1H), 7.40 (t, 1H), 7.25-7.19 (m, 2H), 7.17 (dt, 1H).
LC-MS (Methode 5): $R_t$ = 1.50 min; MS (ESIneg): m/z = 385 $[M-H]^-$.

**Beispiel 21A**

5-(3-Chlorphenyl)-4-(3-cyanphenyl)thiophen-2-carbonsäure

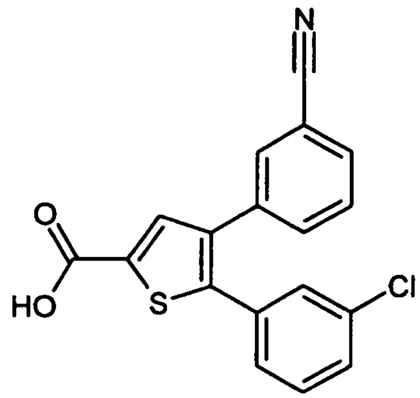

182 mg (0.50 mmol) der Verbindung aus Beispiel 17A werden in 2 ml Dioxan vorgelegt und mit 4.4 ml (8.80 mmol) einer 2N wässrigen Lithiumhydroxid-Lösung versetzt. Man rührt 2 Stunden bei 50°C und engt anschließend ein. Der Rückstand wird mit Wasser verdünnt, mit konzentrierter wässriger HCl-Lösung bis zu einem sauren pH-Wert versetzt und mit Dichlormethan extrahiert. Die organische Phase wird über Magnesiumsulfat getrocknet, filtriert und eingeengt. Das Rohprodukt wird über präparative HPLC (RP18-Säule; Eluent: Acetonitril/Wasser-Gradient) gereinigt. Man erhält 159 mg (95% d. Th.) der Titelverbindung.
$^1$H-NMR (400 MHz, DMSO-$d_6$): $\delta$ = 13.5 (s, 1H), 7.91 (s, 1H), 7.84-7.77 (m, 2H), 7.57-7.51 (m, 2H), 7.50-7.45 (m, 1H), 7.41 (t, 1H), 7.36 (t, 1H), 7.24 (dt, 1H).
LC-MS (Methode 9): $R_t$ = 2.31 min; MS (ESIneg): m/z = 338 $[M-H]^-$.

**Beispiel 22A**

5-(3-Chlor-5-fluorphenyl)-4-(3-cyan-4-fluorphenyl)thiophen-2-carbonsäure

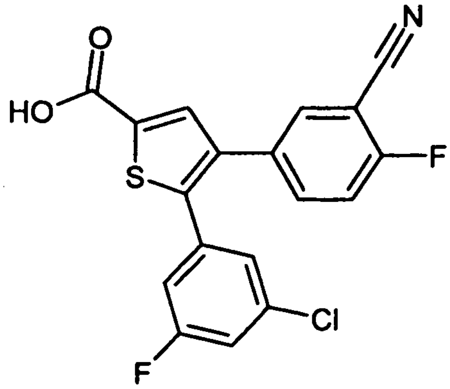

Die Herstellung der Titelverbindung erfolgt ausgehend von der Verbindung aus Beispiel 18A analog zur Synthese der Verbindung aus Beispiel 19A. Man erhält 82.3 mg (97% d. Th.) der Titelverbindung.
$^1$H-NMR (400 MHz, DMSO-$d_6$): $\delta$ = 13.5 (br. s, 1H), 7.99 (dd, 1H), 7.93 (s, 1H), 7.58 (ddd, 1H), 7.55-7.47 (m, 2H), 7.24 (t, 1H), 7.17 (ddd, 1H).
LC-MS (Methode 1): $R_t$ = 2.75 min; MS (ESIpos): m/z = 376 $[M+H]^+$.

**Beispiel 23A**

4-Brom-5-(3-chlorphenyl)thiophen-2-carbonsäure

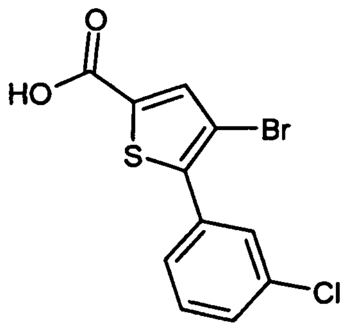

Die Herstellung der Titelverbindung erfolgt ausgehend von der Verbindung aus Beispiel 10A analog zur Synthese der Verbindung aus Beispiel 19A. Man erhält 1.11 g (100% d. Th.) der Titelverbindung.
$^1$H-NMR (400 MHz, DMSO-$d_6$): $\delta$ = 13.7 (s, 1H), 7.78 (s, 1H), 7.75-7.72 (m, 1H), 7.63 (dt, 1H), 7.61-7.54 (m, 2H).
LC-MS (Methode 7): $R_t$ = 2.13 min; MS (ESIneg): m/z = 317 $[M-H]^-$.

**Beispiel 24A**

1-{[4-Brom-5-(3-chlorphenyl)thiophen-2-yl]carbonyl}imidazolidin-4-on

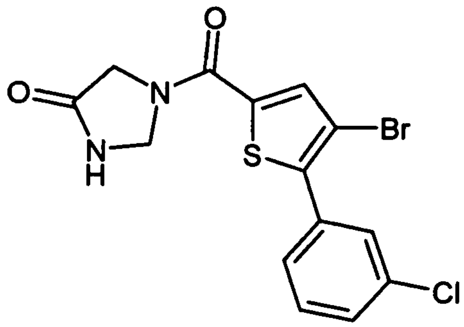

334 mg (1.05 mmol) der Verbindung aus Beispiel 23A, 332 mg (1.16 mmol) 4-Imidazolidinon und 821 mg (1.58 mmol) PyBOP werden in 7.2 ml Tetrahydrofuran vorgelegt und bei Raumtemperatur mit 0.4 ml (2.21 mmol) N,N-Diisopropylethylamin versetzt. Man rührt bei Raumtemperatur über Nacht und reinigt anschließend die Reaktionsmischung über präparative HPLC (RP18-Säule; Eluent: Acetonitril/Wasser-Gradient). Man erhält 226 mg (56% d. Th.) der Titelverbindung.
$^1$H-NMR (400 MHz, DMSO-$d_6$): $\delta$ = 8.78 (s, 1H), 7.83 (s, 1H), 7.74 (s, 1H), 7.67-7.52 (m, 3H), 5.24 (s, 0.5H), 4.87 (s, 1.5H), 4.41 (s, 1.5H), 3.97 (s, 0.5H).
LC-MS (Methode 1): $R_t$ = 2.35 min; MS (ESIpos): m/z = 387 $[M+H]^+$.

**Ausführungsbeispiele:**

**Beispiel 1**

3-{2-(3-Chlor-5-fluorphenyl)-5-[(4-oxoimidazolidin-1-yl)carbonyl]thiophen-3-yl}benzolcarbonitril

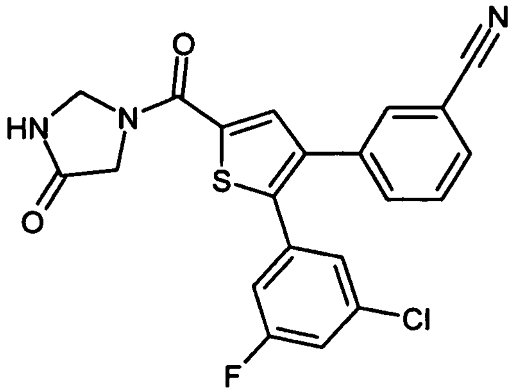

40.5 mg der Verbindung aus Beispiel 19A mit 76%iger Reinheit (0.08 mmol), 24.9 mg (0.13 mmol) der Verbindung aus Beispiel 4A und 88.4 mg (0.17 mmol) PyBOP werden in 2 ml Tetrahydrofuran vorgelegt und bei Raumtemperatur mit 0.04 ml (0.24 mmol) N,N-Diisopropylethylamin versetzt. Man rührt bei Raumtemperatur über Nacht und reinigt anschließend die Reaktionsmischung über präparative HPLC (RP18-Säule; Eluent: Acetonitril/Wasser-Gradient unter Zusatz von 0.1% Ameisensäure). Man erhält 16.8 mg (46% d. Th.) der Titelverbindung.
$^1$H-NMR (400 MHz, DMSO-$d_6$): $\delta$ = 8.85-8.76 (m, 1H), 7.98 (s, 1H), 7.90 (s, 1H), 7.84-7.80 (m, 1H), 7.55-7.49 (m, 3H), 7.22-7.20 (m, 1H), 7.15 (ddd, 1H), 5.31 (s, 0.5H), 4.89 (s, 1.5H), 4.48 (s, 1.5H), 3.99 (s, 0.5H).
LC-MS (Methode 1): $R_t$ = 2.37 min; MS (ESIpos): m/z = 426 $[M+H]^+$.

**Beispiel 2**

1-{[4-(3-Chlor-4-fluorphenyl)-5-(3-chlor-5-fluorphenyl)thiophen-2-yl]carbonyl}imidazolidin-4-on

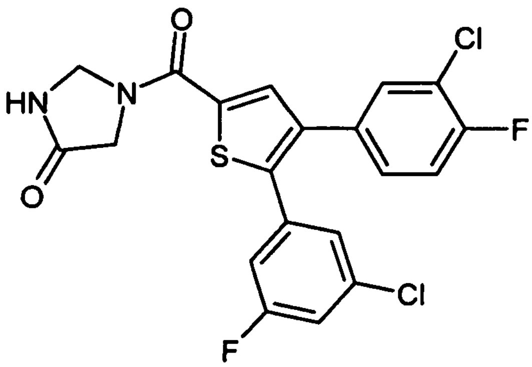

Die Herstellung der Titelverbindung erfolgt ausgehend von 49.1 mg (0.13 mmol) der Verbindung aus Beispiel 20A analog zur Synthese der Verbindung aus Beispiel 1. Man erhält 40.9 mg (71% d. Th.) der Titelverbindung.
$^1$H-NMR (400 MHz, DMSO-$d_6$): $\delta$ = 8.85-8.75 (m, 1H), 7.86 (s, 0.75H), 7.75 (s, 0.25H), 7.72-7.65 (m, 1H), 7.50 (dt, 1H), 7.40 (t, 1H), 7.25-7.19 (m, 2H), 7.16 (dt, 1H), 5.30 (s, 0.5H), 4.89 (s, 1.5H), 4.47 (s, 1.5H), 3.98 (s, 0.5H).
LC-MS (Methode 1): $R_t$ = 2.58 min; MS (ESIpos): m/z = 453 $[M+H]^+$.

**Beispiel 3**

3-(2-(3-Chlorphenyl)-5-[(4-oxoimidazolidin-1-yl)carbonyl]thiophen-3-yl)benzolcarbonitril

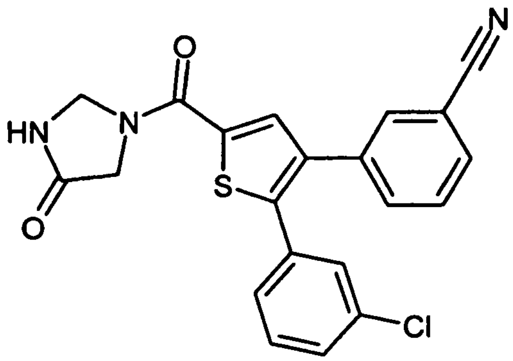

31.3 mg (0.09 mmol) der Verbindung aus Beispiel 21A, 9.5 mg (0.11 mmol) 4-Imidazolidinon und 52.7 mg (0.10 mmol) PyBOP werden in 0.7 ml Tetrahydrofuran vorgelegt und bei Raumtemperatur mit 0.02 ml (0.10 mmol) N,N-Diisopropylethylamin versetzt. Man rührt bei Raumtemperatur über Nacht und reinigt anschließend die Reaktionsmischung über präparative HPLC (RP18-Säule; Eluent: Acetonitril/Wasser-Gradient). Man erhält 15.9 mg (42% d. Th.) der Titelverbindung.
$^1$H-NMR (400 MHz, DMSO-$d_6$): $\delta$ = 8.85-8.75 (m, 1H), 7.97-7.87 (m, 2H), 7.83-7.78 (m, 1H), 7.54-7.50 (m, 2H), 7.47 (d, 1H), 7.41 (t, 1H), 7.36 (s, 1H), 7.24 (d, 1H), 5.31 (s, 0.5H), 4.89 (s, 1.5H), 4.48 (s, 1.5H), 3.99 (s, 0.5H).
LC-MS (Methode 1): $R_t$ = 2.40 min; MS (ESIpos): m/z = 408 $[M+H]^+$.

**Beispiel 4**

5-{2-(3-Chlor-5-fluorphenyl)-5-[(4-oxoimidazolidin-1-yl)carbonyl]thiophen-3-yl}-2-fluorbenzolcarbonitril

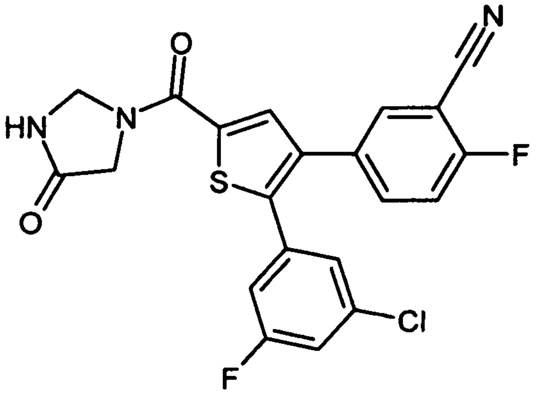

Die Herstellung der Titelverbindung erfolgt ausgehend von 82.3 mg (0.22 mmol) der Verbindung aus Beispiel 22A analog zur Synthese der Verbindung aus Beispiel 1. Man erhält 35.0 mg (35% d. Th.) der Titelverbindung.
$^1$H-NMR (400 MHz, DMSO-$d_6$): δ = 8.87-8.76 (m, 1H), 8.13-8.07 (m, 1H), 7.91-7.75 (m, 1H), 7.60-7.47 (m, 3H), 7.25 (s, 1H), 7.17 (d, 1H), 5.30 (s, 0.5H), 4.89 (s, 1.5H), 4.47 (s, 1.5H), 3.98 (s, 0.5H).
LC-MS (Methode 1): $R_t$ = 2.39 min; MS (ESIpos): m/z = 444 $[M+H]^+$.

**Beispiel 5**

1-{[5-(3-Chlor-5-fluorphenyl)-4-(3-chlorphenyl)thiophen-2-yl]carbonyl}imidazolidin-4-on

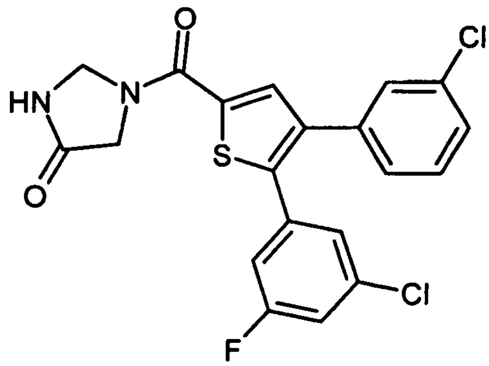

49.0 mg (0.13 mmol) der Verbindung aus Beispiel 16A und 33.2 mg (0.19 mmol) 3-Chlor-5-fluorphenylboronsäure werden unter Argon in 1.8 ml 1,2-Dimethoxyethan vorgelegt und mit 0.7 ml (0.64 mmol) wässriger Natriumhydrogencarbonat-Lösung (10%ig) und 4.4 mg (0.004 mmol) Tetrakis(triphenylphosphin)palladium(0) versetzt. Es wird über Nacht bei 80°C gerührt. Die Reaktionsmischung wird über präparative HPLC (RP18-Säule; Eluent: Acetonitril/Wasser-Gradient unter Zusatz von 0.1% Ameisensäure) gereinigt. Man erhält 12.0 mg (22% d. Th.) der Titelverbindung.
$^1$H-NMR (400 MHz, DMSO-$d_6$): δ = 8.78 (s, 1H), 7.88-7.70 (m, 1H), 7.53-7.47 (m, 2H), 7.43 (dt, 1H), 7.38 (t, 1H), 7.22-7.18 (m, 2H), 7.14 (dt, 1H), 5.31 (s, 0.5H), 4.89 (s, 1.5H), 4.48 (s, 1.5H), 3.99 (s, 0.5H).
LC-MS (Methode 7): $R_t$ = 2.13 min; MS (ESIpos): m/z = 435 $[M+H]^+$.

**Beispiel 6**

5-{3-(3-Chlorphenyl)-5-[(4-oxoimidazolidin-1-yl)carbonyl]thiophen-2-yl}-2-fluorbenzolcarbonitril

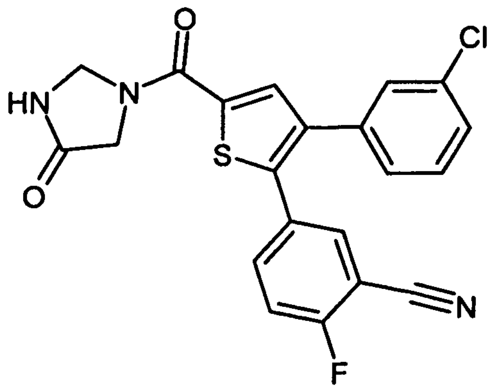

70.0 mg (0.15 mmol) der Verbindung aus Beispiel 16A und 36.1 mg (0.22 mmol) 3-Cyan-4-fluorphenylboronsäure werden unter Argon in 2.1 ml 1,2-Dimethoxyethan vorgelegt und mit 0.8 ml (0.73 mmol) wässriger Natriumhydrogencarbonat-Lösung (10%ig) und 5.1 mg (0.004 mmol) Tetrakis(triphenylphosphin)palladium(0) versetzt. Es wird über Nacht bei 80°C gerührt. Die Reaktionsmischung wird über die präparative HPLC (RP18-Säule; Eluent: Acetonitril/Wasser-Gradient unter Zusatz von 0.1% Ameisensäure) und zusätzlich präparative Dünnschichtchromatographie (Kieselgel; Laufmittel Dichlormethan/Methanol 10/1) gereinigt. Man erhält 5.3 mg (9% d. Th.) der Titelverbindung.
$^1$H-NMR (400 MHz, DMSO-$d_6$): $\delta$ = 8.78 (s, 1H), 7.87-7.71 (m, 1H), 7.53-7.47 (m, 1H), 7.43 (dt, 1H), 7.38 (t, 1H), 7.31 (tt, 1H), 7.20 (d, 1H), 7.06-6.99 (m, 2H), 5.31 (s, 0.5H), 4.89 (s, 1.5H), 4.48 (s, 1.5H), 3.99 (s, 0.5H).
LC-MS (Methode 7): $R_t$ = 1.88 min; MS (ESIpos): m/z = 426 $[M+H]^+$.

**Beispiel 7**

1-({4-(3-Chlorphenyl)-5-[3-(trifluormethoxy)phenyl]thiophen-2-yl}carbonyl)imidazolidin-4-on

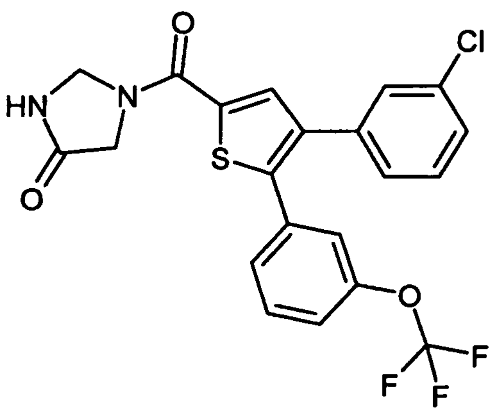

Die Herstellung der Titelverbindung erfolgt ausgehend von 70.0 mg (0.15 mmol) der Verbindung aus Beispiel 16A und 45.1 mg (0.22 mmol) 3-(Trifluormethoxy)phenylboronsäure analog zur Synthese der Verbindung aus Beispiel 5. Man erhält 26.7 mg (39% d. Th.) der Titelverbindung.
$^1$H-NMR (400 MHz, DMSO-$d_6$): $\delta$ = 8.78 (s, 1H), 7.86 (s, 0.75H), 7.74 (s, 0.25H), 7.56 (t, 1H), 7.46-7.33 (m, 5H), 7.21 (d, 1H), 7.14 (s, 1H), 5.31 (s, 0.5H), 4.89 (s, 1.5H), 4.48 (s, 1.5H), 3.99 (s, 0.5H).
LC-MS (Methode 7): $R_t$ = 2.17 min; MS (ESIpos): m/z = 467 $[M+H]^+$.

**Beispiel 8**

5-{2-(3-Chlorphenyl)-5-[(4-oxoimidazolidin-1-yl)carbonyl]thiophen-3-yl}-2-fluorbenzolcarbonitril

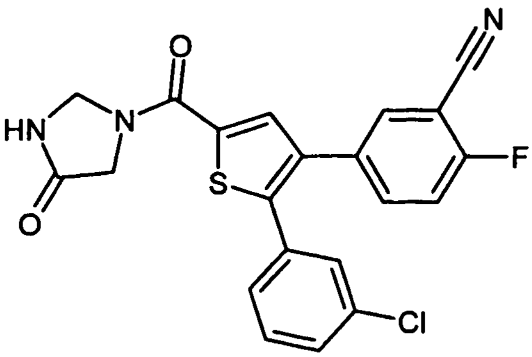

61.0 mg (0.16 mmol) der Verbindung aus Beispiel 24A werden unter Argon in 3 ml 1,2-Dimethoxyethan vorgelegt und mit 26.1 mg (0.16 mmol) 3-Cyan-4-fluorphenylboronsäure, 155 mg (0.47 mmol) Cäsiumcarbonat, 5.3 mg (0.01 mmol) Dicyclohexyl[2',4',6'-tri(propan-2-yl)biphenyl-2-yl]phosphan und 1.1 mg (0.005 mmol) Palladium(II)acetat versetzt. Es wird 30 min bei 100°C unter Mikrowellenbestrahlung in einem geschlossenen Glasgefäß erhitzt. Die Reaktionsmischung wird über die präparative HPLC (RP18-Säule; Eluent: Acetonitril/Wasser-Gradient) gereinigt. Man erhält 23.0 mg (34% d. Th.) der Titelverbindung.

$^1$H-NMR (400 MHz, DMSO-$d_6$): $\delta$ = 8.86-8.74 (m, 1H), 8.09-8.02 (m, 1H), 7.90-7.73 (m, 1H), 7.56 (ddd, 1H), 7.52-7.46 (m, 2H), 7.44-7.38 (m, 2H), 7.23 (d, 1H), 5.31 (s, 0.5H), 4.89 (s, 1.5H), 4.48 (s, 1.5H), 3.99 (s, 0.5H).

LC-MS (Methode 1): $R_t$ = 2.50 min; MS (ESIpos): m/z = 426 $[M+H]^+$.

**Beispiel 9**

1-{[4-(3-Chlor-4-fluorphenyl)-5-(3-chlorphenyl)thiophen-2-yl]carbonyl}imidazolidin-4-on

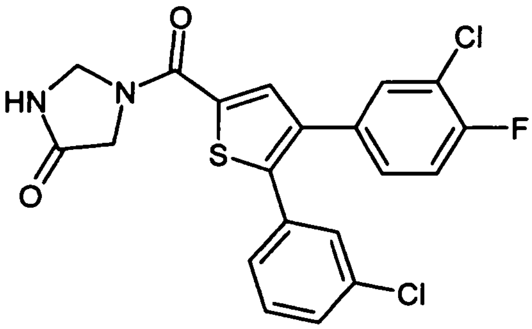

70.0 mg der Verbindung aus Beispiel 24A mit 76%iger Reinheit (0.14 mmol) und 47.5 mg (0.27 mmol) 3-Chlor-4-fluorphenylboronsäure werden unter Argon in 2.6 ml 1,2-Dimethoxyethan vorgelegt und mit 1.0 ml (0.91 mmol) wässriger Natriumhydrogencarbonat-Lösung (10%ig) und 6.3 mg (0.005 mmol) Tetrakis(triphenylphosphin)palladium(0) versetzt. Es wird 5 min bei 150°C unter Mikrowellenbestrahlung in einem geschlossenen Glasgefäß erhitzt. Die Reaktionsmischung wird über präparative HPLC (RP18-Säule; Eluent: Acetonitril/Wasser-Gradient unter Zusatz von 0.1% Ameisensäure) und zusätzlich präparative Dünnschichtchromatographie (Kieselgel; Laufmittel Essigsäureethylester) gereinigt. Man erhält 8.0 mg (13% d. Th.) der Titelverbindung.

$^1$H-NMR (400 MHz, DMSO-$d_6$): $\delta$ = 8.82-8.75 (m, 1H), 7.87-7.71 (m, 1H), 7.69-7.63 (m, 1H), 7.49-7.35 (m, 4H), 7.27-7.18 (m, 2H), 5.30 (s, 0.5H), 4.88 (s, 1.5H), 4.47 (s, 1.5H), 3.98 (s, 0.5H).

LC-MS (Methode 1): $R_t$ = 2.54 min; MS (ESIpos): m/z = 435 $[M+H]^+$.

**Beispiel 10**

3-{3-(3-Chlor-4-fluorphenyl)-5-[(4-oxoimidazolidin-1-yl)carbonyl]thiophen-2-yl}-5-fluorbenzolcarbonitril

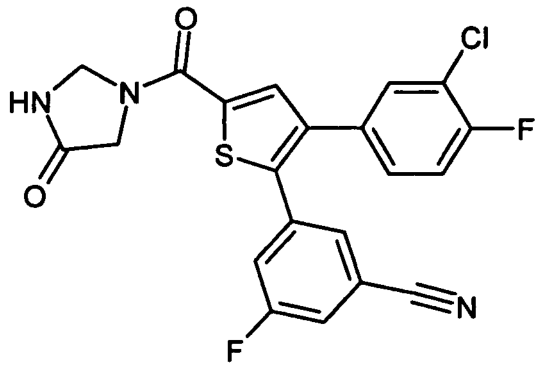

1.50 g (4.78 mmol) der Verbindung aus Beispiel 8A werden unter Argon in 75 ml 1,2-Dimethoxyethan vorgelegt und mit 1.30 g (5.26 mmol) der Verbindung aus Beispiel 5A, 4.67 g (14.3 mmol) Cäsiumcarbonat, 159 mg (0.33 mmol) Dicyclohexyl[2',4',6'-tri(propan-2-yl)biphenyl-2-yl]phosphan und 32.0 mg (0.14 mmol) Palladium(II)acetat versetzt. Es wird über Nacht bei 50°C gerührt. Anschließend gibt man Wasser hinzu, extrahiert mit Essigsäureethylester, trocknet über Natriumsulfat, filtriert und engt ein. Das Rohprodukt wird über präparative HPLC (RP18-Säule; Eluent: Acetonitril/Wasser-Gradient unter Zusatz von 0.1% Ameisensäure) gereinigt. Man erhält 1.05 g 4-Brom-5-(3-cyan-5-fluorphenyl)thiophen-2-carbonsäureethylester.
LC-MS (Methode 1): $R_t$ = 2.91 min; MS (ESIpos): m/z = 354 $[M+H]^+$.
Die Hälfte des so erhaltenen Feststoffs und 517 mg (2.96 mmol) 3-Chlor-4-fluorphenylboronsäure werden unter Argon in 21 ml 1,2-Dimethoxyethan vorgelegt und mit 7.9 ml (7.41 mmol) wässriger Natriumhydrogencarbonat-Lösung (10%ig) und 51.0 mg (0.04 mmol) Tetrakis(triphenylphosphin)palladium(0) versetzt. Es wird über Nacht bei 80°C gerührt. Anschließend engt man ein, gibt 1N wässrige HCl-Lösung bis zu einem sauren pH-Wert zu, extrahiert mit Essigsäureethylester, trocknet über Natriumsulfat, filtriert und engt ein. Das Rohprodukt wird über präparative HPLC (RP18-Säule; Eluent: Acetonitril/Wasser-Gradient unter Zusatz von 0.1% Ameisensäure) gereinigt. Man erhält 41.7 mg 4-(3-Chlor-4-fluorphenyl)-5-(3-cyan-5-fluorphenyl)thiophen-2-carbonsäureethylester.
GC-MS (Methode 11): $R_t$ = 9.26 min; MS (EIpos): m/z = 403 $[M]^+$.
Der so erhaltene Feststoff wird in 1.8 ml Tetrahydrofuran vorgelegt und bei Raumtemperatur mit 24.7 mg (1.03 mmol) Lithiumhydroxid und 0.6 ml Wasser versetzt. Man rührt bei Raumtemperatur über Nacht, gibt anschließend 1N wässrige HCl-Lösung bis zu einem sauren pH-Wert hinzu, extrahiert mit Essigsäureethylester, trocknet über Natriumsulfat, filtriert und engt ein. Man erhält 29.8 mg 4-(3-Chlor-4-fluorphenyl)-5-(3-cyan-5-fluorphenyl)thiophen-2-carbonsäure. Dieser Feststoff, 16.0 mg (0.08 mmol) der Verbindung aus Beispiel 4A und 56.8 mg (0.11 mmol) PyBOP werden in 2 ml Tetrahydrofuran vorgelegt und bei Raumtemperatur mit 0.04 ml (0.23 mmol) N,N-Diisopropylethylamin versetzt. Man rührt bei Raumtemperatur über Nacht und reinigt anschließend die Reaktionsmischung über präparative HPLC (RP18-Säule; Eluent: Acetonitril/Wasser-Gradient unter Zusatz von 0.1% Ameisensäure). Man erhält 7.4 mg (1% d. Th.) der Titelverbindung.
$^1$H-NMR (400 MHz, DMSO-$d_6$): $\delta$ = 8.85-8.76 (m, 1H), 7.91 (ddd, 1H), 7.88 (s, 0.75H), 7.76 (s, 0.25H), 7.74-7.67 (m, 2H), 7.54 (dt, 1H), 7.39 (t, 1H), 7.19 (ddd, 1H), 5.31 (s, 0.5H), 4.89 (s, 1.5H), 4.48 (s, 1.5H), 3.99 (s, 0.5H).
LC-MS (Methode 5): $R_t$ = 1.21 min; MS (ESIpos): m/z = 444 $[M+H]^+$.

**Beispiel 11**

3-{3-(3-Chlorphenyl)-5-[(4-oxoimidazolidin-1-yl)carbonyl]thiophen-2-yl}-5-fluorbenzolcarbonitril

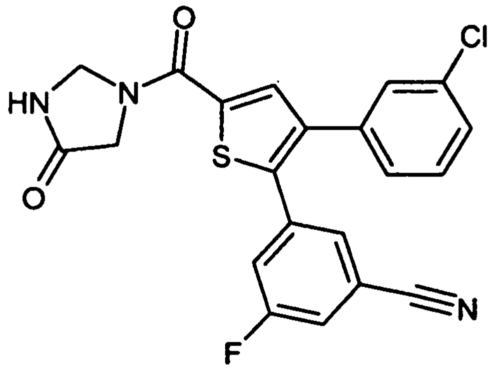

Die Herstellung der Titelverbindung erfolgt ausgehend von der Verbindung aus Beispiel 8A analog zur Synthese der Verbindung aus Beispiel 10. Man erhält 8.2 mg (1% d. Th.) der Titelverbindung.
$^1$H-NMR (400 MHz, DMSO-$d_6$): $\delta$ = 8.83-8.76 (m, 1H), 7.90 (ddd, 1H), 7.89-7.74 (m, 1H), 7.66 (s, 1H), 7.55-7.49 (m, 2H), 7.43 (ddd, 1H), 7.36 (t, 1H), 7.17 (d, 1H), 5.31 (s, 0.5H), 4.89 (s, 1.5H), 4.48 (s, 1.5H), 3.99 (s, 0.5H).
LC-MS (Methode 5): $R_t$ = 1.20 min; MS (ESIpos): m/z = 426 $[M+H]^+$.

**B) Bewertung der physiologischen Wirksamkeit**

**Abkürzungen:**

| | |
|---|---|
| DMSO | Dimethylsulfoxid |
| FKS | Fötales Kälber Serum (Biochrom AG, Berlin, Deutschland) |
| PBS | Phosphate buffered saline |
| MTP | Mikrotiterplatte |
| ELISA | Enzyme-linked immunosorbent assay |

Die Eignung der erfindungsgemäßen Verbindungen zur Behandlung von durch Retroviren hervorgerufenen Erkrankungen kann in folgenden Assay-Systemen gezeigt werden:

**In vitro Assays**

**Biochemischer Reverse Transkriptase Assay**

Es wird der "Reverse Transcriptase Assay, colorimetric" (Roche Diagnostics GmbH, Mannheim, Deutschland) entsprechend den Herstellerangaben verwendet. Die Prüfsubstanzen werden in DMSO gelöst und in 5er Schritten verdünnt in dem Test eingesetzt (DMSO Endkonzentration 1%). Die resultierenden Werte der photometrischen Auswertung (405/492 nm) sind bei der Negativkontrolle (Ansatz ohne Reverse Transkriptase) kleiner 0,1 und liegen bei der Positivkontrolle (Ansatz ohne Prüfsubstanz) im Bereich von 1,5. Die $IC_{50}$-Werte der Prüfsubstanzen werden als die Konzentration der Prüfsubstanzverdünnung ermittelt, bei der die gemessene optische Dichte 50% der Positivkontrolle beträgt.
Es wird gefunden, dass die erfindungsgemäßen Verbindungen die Reverse Transkriptase Aktivität hemmen. Experimentelle Daten sind in Tabelle A zusammengefasst.

**Light Assay mit wildtyp und Inhibitor-resistenten HI-Reporterviren**

Für diesen Assay werden HIV-$1_{NL4-3}$ Reporterviren verwendet, die anstelle des *nef* Gens das *lu164* Gen (Luziferase 164) tragen. Die Viren werden durch Transfektion von 293T-Zellen mit dem entsprechenden proviralen pNL4-3 Plasmid generiert (Lipofectamine Reagent, Invitrogen, Karlsruhe, Deutschland). Ausgehend von der proviralen Plasmid-DNA werden mit dem "QuikChange II XL Site-Directed Mutagenesis Kit" (Stratagene, Cedar Creek, Texas, USA) Viren mit definierten Resistenzmutationen im Reverse Transkriptase Gen hergestellt. Es werden u.a. folgende Mutationen generiert: A98G, A98G-K103N-V108I, A98S, F227C, F227L, G190A, G190S, K101E, K101Q-K103N, K103N, K103N-F227L, K103N-G190A, K103N-G190S, K103N-M230L, K103N-N348I, K103N-P225H, K103N-V108I, K103N-V108I-P225H, K103N-V179F-Y181C, K103N-Y181C, K103N-Y181C-G190A, L100I, L100I-K103N, L100I-K103N-V179I-Y181C, L100I-K103N-Y181C, L234I, N348I, P225H, P236L, V106A, V106A-E138K, V106A-F227C, V106A-F227L, V106I, V106I-Y188L, V106M, V108I, V179F-Y181C, V179I, V179I-Y181C, Y181C, Y181C-G190A, Y181C-M230L, Y181I, Y188L. Mit diesen Reporterviren infizierte MT4 7F2 Zellen sekretieren Luziferase ins Medium, was die luminometrische Quantifizierung der Virusreplikation ermöglicht.

Für den Ansatz einer 96-well MTP werden 3 Millionen MT4 7F2 Zellen pelletiert, in 1 ml RPMI 1640 Medium ohne Phenolrot (Invitrogen, Karlsruhe, Deutschland)/10% FKS/10% AIM-V (Invitrogen, Karlsruhe, Deutschland) suspendiert und zusammen mit einer geeigneten Menge des entsprechenden HIV-$1_{NL4-3}$ Reportervirus für 2 Stunden bei 37°C inkubiert (Pelletinfektion). Nicht adsorbierte Viren werden anschließend mit PBS ausgewaschen, die infizierten Zellen wieder pelletiert und in 8 ml RPMI 1640 Medium ohne Phenolrot/2% oder 10% FKS/10% AIM-V suspendiert. Davon werden 80 µl pro Well in eine weiße 96-well MTP zu 20 µl Prüfsubstanz in geeigneter Verdünnung pipettiert. Um Randeffekte zu vermeiden werden die Randwells der MTP nicht für Substanzverdünnungen verwendet. Die zweite vertikale Reihe der MTP enthält nur infizierte Zellen (Viruskontrolle) und die elfte vertikale Reihe nur nicht infizierte Zellen (Zellkontrolle) jeweils in RPMI 1640 Medium ohne Phenolrot/2% oder 10% FKS/10% AIM-V. Die übrigen Wells der MTP enthalten die erfindungsgemäßen Verbindungen in unterschiedlichen Konzentrationen ausgehend von der dritten vertikalen Reihe, von der aus die Prüfsubstanzen in 3er Schritten bis zur zehnten vertikalen Reihe $3^7$-fach verdünnt werden. Die Prüfsubstanzen sind in DMSO gelöst, wobei die DMSO Endkonzentration im Testansatz 1% beträgt. Die Testansätze werden 5 Tage bei 37°C/5% $CO_2$ inkubiert und nach Zugabe von 15 µl Lu164-Substrat (5 mg/ml Coelenterazin gelöst in 30 µM Glutathion/DMSO, 100 mM NaCl, 1 M MES, 100 mM Glutathion) luminometrisch ausgewertet. Die resultierenden Werte liegen bei der Viruskontrolle im Bereich von 1.000.000 RLUs (relative Lichteinheiten) und bei der Zellkontrolle bei 300 bis 400 RLUs. Die $EC_{50}$-Werte der Prüfsubstanzen werden als die Konzentration ermittelt, bei der die in RLUs gemessene Virusreplikation 50% der unbehandelten infizierten Zellen beträgt.

Es wird gefunden, dass die erfindungsgemäßen Verbindungen die HIV-Replikation hemmen. Experimentelle Daten sind in Tabelle A zusammengefasst.

**PBL- und H9-Assay mit wildtyp HIV-1**

Primäre menschliche Blutlymphozyten (PBLs) werden über Ficoll-Paque Leucosep Röhrchen (Greiner Bio-One, Frickenhausen, Deutschland) aus Blut isoliert und in RPMI 1640 Medium (Invitrogen, Karlsruhe, Deutschland)/10% FKS mit Phytohaemagglutinin (90 µg/ml) und Interleukin-2 (40 U/ml) 3 Tage stimuliert.

Für den Ansatz einer 96-well MTP werden 3 Millionen PBLs pelletiert, in 1 ml RPMI 1640 Medium/10% FKS suspendiert und zusammen mit einer geeigneten Menge HIV-$1_{LAI}$ (NIH AIDS Research & Reference Reagent Program, Germantown, USA) für 2 Stunden bei 37°C inkubiert (Pelletinfektion). Nicht adsorbierte Viren werden anschließend mit PBS ausgewaschen, die infizierten Zellen wieder pelletiert und in 18ml RPMI 1640 Medium/10% FKS/Interleukin-2 (40U/ml) suspendiert. Davon werden 180 µl pro well in eine weiße 96-well MTP zu 20 µl Prüfsubstanz in geeigneter Verdünnung pipettiert. Alternativ wird das HIV nach Zubereitung der Substanzverdünnungen in der MTP zusammen mit den Zellen zupipettiert und wird nicht mehr ausgewaschen (Überstandsinfektion). Um Randeffekte zu vermeiden werden die Randwells der MTP nicht für Substanzverdünnungen verwendet. Die zweite vertikale Reihe der MTP enthält nur infizierte Zellen (Viruskontrolle) und die elfte vertikale Reihe nur nicht infizierte Zellen (Zellkontrolle) jeweils in RPMI 1640 Medium/10% FKS/Interleukin-2 (40 U/ml). Die übrigen Wells der MTP enthalten die erfindungsgemäßen Verbindungen in unterschiedlichen Konzentrationen ausgehend von der dritten vertikalen Reihe, von der aus die Prüfsubstanzen in 3er Schritten bis zur zehnten vertikalen Reihe $3^7$-fach verdünnt werden. Die Prüfsubstanzen sind in DMSO gelöst, wobei die DMSO Endkonzentration im Testansatz 1% beträgt. Die Testansätze werden bei 37°C/5% $CO_2$ inkubiert. Nach 5 und 7 Tagen erfolgt die Abnahme von jeweils 50µl zellfreiem Überstand aus jedem Well zur Bestimmung der enthaltenen p24 Menge mittels p24 ELISA (HIV-1 $p24^{CA}$ Antigen Capture Assay Kit, NCI-Frederick Cancer Research and Development Center, Frederick, USA). Aus den resultierenden Werten der photometrischen Auswertung (450/620 nm) werden die $EC_{50}$-Werte der Prüfsubstanzen als die Konzentration ermittelt, bei der die p24 Menge 50% der unbehandelten infizierten Zellen beträgt.

Alternativ werden H9-Zellen (ATCC, Wesel, Deutschland) anstelle von PBLs zum Test der Prüfsubstanzen eingesetzt. H9-Zellen werden nach oben beschriebenem Muster in RPMI 1640 Medium mit 2% oder 10% FKS als HIV-$1_{LAI}$ Überstandsinfektion 5 Tage bei 37°C/5% $CO_2$ inkubiert (20 µl Substanzverdünnung und 80 µl Zellen/Virus pro Well). Anschließend wird je Well 10 µl AlamarBlue (Invitrogen, Karlsruhe, Deutschland) zugegeben und die MTPs werden für 3 Stunden bei 37°C inkubiert, bevor die fluorimetrische Auswertung erfolgt (544/590 nm). Die resultierenden Werte liegen bei den unbehandelten nicht infizierten Zellen bei etwa 40.000 und bei den unbehandelten infizierten Zellen bei etwa 7.000. Im niedrigen Konzentrationsbereich werden die $EC_{50}$-Werte der Prüfsubstanzen als die Konzentration ermittelt, bei der die Fluoreszenz 50% der unbehandelten nicht infizierten Zellen beträgt (jeweils abzüglich der Werte der unbehandelten infizierten Zellen). Außerdem werden im hohen Konzentrationsbereich die $CC_{50}$-Werte der Prüfsubstanzen als die Konzentration ermittelt, bei der die Fluoreszenz 50% der unbehandelten nicht infizierten Zellen beträgt

(jeweils abzüglich der Werte der unbehandelten infizierten Zellen).

Es wird gefunden, dass die erfindungsgemäßen Verbindungen die HIV-Replikation hemmen. Experimentelle Daten sind in Tabelle A zusammengefasst.

**Assay zur Bestimmung der zytotoxischen Wirkung der Prüfsubstanzen**

Zur Bestimmung der zytotoxischen Wirkung der Prüfsubstanzen in nicht infizierten Zellen werden die Substanzen in entsprechenden Konzentrationen auf durchsichtige 96-well MTPs pipettiert und mit nicht infizierten Zellen (z.B. H9, PBLs, THP-1, MT4 7F2, CEM, Jurkat) inkubiert (analog zu den oben beschriebenen Assays). Nach 5 Tagen wird zu den Testansätzen je Well 1/10 Volumen AlamarBlue zugegeben und die MTPs werden für 3 Stunden bei 37°C inkubiert. Anschließend erfolgt die fluorimetrische Auswertung (544/590 nm). Die resultierenden Werte liegen bei nicht behandelten Zellen je nach Zellart zwischen 20.000 und 40.000. Die $CC_{50}$-Werte der Prüfsubstanzen werden als die Konzentration ermittelt, bei der die Fluoreszenz 50% der unbehandelten Zellen beträgt. Prüfsubstanzen, die im Konzentrationsbereich der Wirkung zytotoxische Befunde zeigen, werden nicht in ihrer antiviralen Wirksamkeit bewertet.

**Tabelle A:**

| Beispiel-Nr. | $IC_{50}$ (nM) RT Assay | $EC_{50}$ (nM) H9 Zellen HIV-$1_{LAI}$ 10% FKS | $EC_{50}$ (nM) MT4 ZF2 Zellen HIV-$1_{NLA-3}$ wt 2% FKS | $EC_{50}$ (nM) MT4 7F2 Zellen HIV-$1_{NL4-3}$ K103N-Y181C 2% FKS |
|---|---|---|---|---|
| Beispiel 1 | | 9 | 1 | 45 |
| Beispiel 2 | 920 | 68 | 20 | 176 |
| Beispiel 3 | 740 | 71 | 5 | 100 |
| Beispiel 4 | | 12 | <1.5 | 44 |
| Beispiel 5 | 2170 | 85 | 21 | 258 |
| Beispiel 6 | | 900 | 165 | 348 |
| Beispiel 7 | | 1410 | 100 | 150 |
| Beispiel 8 | | 103 | 5 | 121 |
| Beispiel 9 | | 593 | 35 | 260 |
| Beispiel 10 | | 34 | 5 | 40 |
| Beispiel 11 | | 22 | 2 | 123 |

**In vivo Assay**

**Tiermodell:**

NOD Scid Mäuse, in der Regel 5 - 6 Wochen alt, werden von kommerziellen Züchtern (z. B. Taconic oder Jackson Laboratory) bezogen. Die Tiere werden unter sterilen Bedingungen (einschließlich Streu und Futter) in Isolatoren gehalten.

Eine definierte Anzahl von Zellen (z. B. 5 x $10^6$ T-Zellen (z. B. C8166)) wird mit einer geeigneten m.o.i. (z.B. 0.01 $TCID^{50}$) mit HIV infiziert. Die infizierten Zellen werden in Kollagenschwämme eingebracht. Die so vorbehandelten Schwämme werden den Mäusen unter die Rückenhaut implantiert. Die Mäuse werden einmal oder mehrfach täglich peroral, intraperitoneal, subcutan oder intravenös behandelt, wobei die erste Behandlung vor der Implantation liegen kann. Die Behandlungsgruppen umfassen in der Regel 10 Mäuse. Mindestens eine Gruppe wird mit Placebo behandelt, mindestens eine Gruppe mit einer bekanntermaßen wirksamen Substanz (= Positivkontrolle) und in der Regel mehrere Gruppen mit der erfindungsgemäßen Substanz. Die Tagesdosis der erfindungsgemäßen Substanz liegt zwischen 0.01 mg und 100 mg pro kg Körpergewicht. Die Formulierung der Substanzen erfolgt in 2% DMSO/0.5% Methylcellulose in PBS oder einer anderen geeigneten Mischung, die die Löslichkeit der Substanzen unterstützt. Die Behandlungsdauer beträgt in der Regel viereinhalb Tage. Nach der letzten Substanzapplikation werden die Tiere getötet und die Schwämme entnommen. Die virusinfizierten Zellen werden durch Kollagenaseverdau aus dem Schwamm gewonnen.

Aus den Zellen wird Gesamt-RNA gewonnen, die in der quantitativen PCR auf den Gehalt an Virus-RNA überprüft wird. Die Menge an Virus-RNA wird anhand der Menge eines house-keeping Gens (z. B. GAPDH) normalisiert. Ermittelt wird die Menge an HIV-RNA nach Substanzbehandlung im Vergleich zur placebobehandelten Kontrollgruppe.

Wurde ein HIV verwendet, das eine Luziferase trägt, kann zusätzlich oder ersatzweise eine Luziferase-Messung durchgeführt werden. In diesem Fall wird die HIV-Menge über die Höhe des Luziferase-Signals bestimmt, da es in diesem Fall als Maß für die Virusreplikation dient. Die statistische Auswertung erfolgt mittels geeigneter Computerprogramme, z. B. Graph Pad Prism.

**B) Bewertung der pharmakokinetischen Eigenschaften**

***In vivo* Studien**

Zur Bestimmung der *in vivo* Pharmakokinetik werden die Testsubstanzen Mäusen, Ratten und Hunden intravenös und oral appliziert. Bei intravenösen Studien wird zur Bestimmung der pharmakokinetischen Eigenschaften der Testsubstanzen eine Dosis von 0.5 mg/kg in allen Spezies gewählt. Bei oraler Gabe wird den Nagern 3 mg/kg appliziert, Hunden 1 mg/kg. Die Testsubstanzen werden zur intravenösen Gabe für Nager in 99% Plasma, 1% DMSO formuliert, bei oraler Gabe in PEG 400, Ethanol und Wasser in variierenden Anteilen. Letzteres Vehikel wird bei Hunden für beide Applikationsrouten verwendet.

Männliche Wistar Ratten werden vor Applikation der Testsubstanzen katheterisiert, so dass die Blutproben mit Hilfe des gelegten Katheters oder durch Punktion der Vena cava zu verschiedenen Zeitenpunkten über ein Intervall von 2 min bis zu 26 h entnommen werden können.

Weiblichen BalbC Mäusen werden die Testsubstanzen als bolus Injektion intravenös appliziert, die Probengewinnung erfolgt in diesem Falle ausschließlich durch Punktion der Vena cava über ein Intervall von 2 min bis zu 26 h. Bei weiblichen Beagle Hunden erfolgt die Applikation ausschließlich durch eine 15 minütige intravenöse Infusion. Die Proben werden durch Punktion der Vena brachialis oder der Vena jugularis über ein Intervall von 10 min bis zu 26 h gewonnen.

Die quantitative Bestimmung der Substanzen erfolgt aus dem gewonnenen Tierplasma und Eichproben, die in Plasma eingestellt werden. Die Plasmaproteine werden durch Fällung mit Acetonitril (ACN) entfernt. Anschließend werden die Proben mittels HPLC auf einer Agilent 1100 LC Anlage (Agilent, Santa Clara, Californien, USA) unter Verwendung unterschiedlicher Säulen, z.B. Luna C8, LichroCart Purospher Star RP18e aufgetrennt. Das HPLC System ist über ein Turbo Ion Spray Interface an ein Triple Quadropole Massenspektrometer API 3000 (Applied Biosystems, Darmstadt, Deutschland) gekoppelt. Die Auswertung des Plasmakonzentrations-Zeitverlaufs erfolgt unter Einsatz eines internen Standards und Verwendung eines validierten Kinetikauswerteprogramms.

Neben Studien zur Bestimmung der pharmakokinetischen Parameter der Testsubstanzen *in vivo* werden Bestimmungen der relativen Bioverfügbarkeit aus Suspension (Formulierung: Tylose Suspension) versus Lösung in der Ratte sowie Hochdosisstudien im Vorfeld von Wirkversuchen und toxikologischen Studien in Mäusen, Ratten und Hunden durchgeführt.

**Plasmastabilität**

Das verwendete Plasma der unterschiedlichen Spezies (BalbC Maus, Wistar Ratte, Beagle Hund und Mensch) wird durch Blutabnahme, in mit Li-Heparin beschichtete Monovetten und anschließender Zentrifugation, frisch gewonnen. Zur Bestimmung der Plasmastabilität der Testsubstanzen wird je 500 ng/ml bei 37°C, zu je 2 ml in Plasma inkubiert. Zu verschiedenen Zeitpunkten, über ein Intervall bis zu 3 h, werden Proben dem Inkubationsgefäß entnommen. Die gewonnenen Proben werden mit ACN gefällt, um die Umsetzung zu stoppen und die Plasmaproteine abzutrennen. Die Proben werden äquivalent zu den *in vivo* Studien analysiert.

**Mikrosomale und Hepatozyten Inkubationen**

Inkubationen mit Lebermikrosomen verschiedener Spezies (BalbC Maus, Wistar Ratte, Beagle Hund, Mensch) werden in einem Gesamtvolumen von 1.5 ml bei 37°C auf einem modifizierten Multiprobe II® Robotersytem (Canberra Packard) bzw. Janus® Robotersystem (Perkin Elmer) durchgeführt.

Die Inkubationsmischungen enthalten jeweils 0.5 µg/ml Testsubstanz sowie 0.2 - 0.5 mg/ml mikrosomales Protein. Zusätzlich wird 0.05 M Phosphatpuffer (pH = 7.4), 1 mM EDTA, 5 mM Glucose-6-phosphat und 1.5 U/ml Glucose-6-phosphat Dehydroxygenase aus *Leuconostoc Mesenteroides* zugesetzt. Die mikrosomale Inkubationen wird durch Zugabe von $NADP^+$ (Endkonzentration: 1 mM) gestartet.

Zur Bestimmung der metabolischen Stabilität der Testsubstanzen in frisch isolierten und kultivierten Ratten-Hunde-, und Humanhepatozyten werden jeweils 1 Millionen Zellen/ml verwendet. Äquivalent dem mikrosomalen Assay werden den Hepatozyten jeweils 0.5 µg/ml Testsubstanz zugesetzt.

Nach 2, 5, 10, 20, 30, 45 und 60 min, oder nach 2, 10, 20, 30, 50, 70 und 90 min bei stabileren Verbindungen, werden 125 µl aus dem jeweiligen Inkubationsansatz entnommen und mit ACN versetzt, um die enzymatischen Reaktionen zu stoppen. Nach der Zentrifugation werden die Proben mittels LC-MS/MS (API 2000 oder 3000, Applied Biosystems) analysiert. "$CL_{blood}$ well-stirred"- und "$F_{max}$ well-stirred"-Werte werden aus den jeweiligen Halbwertszeiten der Verbindungen in den mikrosomalen Inkubationen errechnet. Der Substratabbau kann mit folgenden Formeln beschrieben werden (Houston JB, Utility of in-vitro drug-metabolism data in predicting in-vivo metabolic-clearance, Bioch. Pharm. 47 (9) 1469- 1479 (1994); Obach RS; Baxter JG; Liston TE; Silber BM; Jones BC; MacIntyre F; Rance DJ; Wastall P, The prediction of human pharmacokinetic parameters from preclinical and in vitro metabolism data, J. Pharmacol. Exp. Ther. 283 (1) 46- 58 (1997)):

$$CL'_{intrinsic}\ [ml/(min\cdot kg)] = (0.693/\text{in vitro } t_{1/2}\ [min]) \cdot (\text{Lebergewicht } [g \text{ Leber/kg Körpergewicht}]) \cdot (\text{mikrosomales Protein } [mg]/\text{Lebergewicht } [g])/(\text{mikrosomales Protein } [mg]/\text{Inkubationsvolumen } [ml])$$

Die Blut-Clearance "$Cl_{blood}$" wird ohne die Berücksichtigung von Proteinbindungen durch das "well-stirred" Modell beschrieben (Pang KS; Rowland M, Hepatic clearance of drugs. I. Theoretical considerations of a "well-stirred" model and a "parallel tube" model. Influence of hepatic blood flow, plasma and blood cell binding, and the hepatocellular enzymatic activity on hepatic drug clearance, J Pharmacokinet Biopharm 5 (6): 625-53 (1977)):

$$CL_{blood}\ \text{well-stirred}\ [l/(h\cdot kg)] = (Q_H\ [l/(h\cdot kg)] \cdot CL'_{intrinsic}\ [l/(h\cdot kg)])/(Q_H\ [l/(h\cdot kg)] + CL'_{intrinsic}\ [l/(h\cdot kg)])$$

Für die Ratten beträgt das spezifische Lebergewicht 32 g/kg Körpergewicht und der hepatische Blutfluss 4.2 1/(h·kg). Der spezifische mikrosmale Proteingehalt der Rattenleber wurde mit 40 mg/g Leber veranschlagt. Die spezifischen Hochrechnungsfaktoren weiterer Spezies sind in folgender Tabelle wiedergegeben und beruhen zum Teil auf Literaturdaten, zum Teil auf eigenen Bestimmungen. Für Hepatozyten wird eine Zellzahl von 110 Mio/g Leber bei allen Spezies zur Berechnung herangezogen.

| | Maus m | Maus w | Ratte m | Hund m/w | Mensch m/w |
|---|---|---|---|---|---|
| Mikrosomales Protein/g Leber [mg] | 40 | 40 | 40 | 40 | 40 |
| Leber [g]/kg Körpergewicht | 50 | 43 | 32 | 39 | 21 |
| Leberblutfluss [1/(h·kg)] | 5.4 | 5.4 | 4.2 | 2.1 | 1.32 |

**C) <u>Ausführungsbeispiele für pharmazeutische Zusammensetzungen</u>**

Die erfindungsgemäßen Verbindungen können folgendermaßen in pharmazeutische Zubereitungen überführt werden:

**Tablette:**

Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

Herstellung:

Die Mischung aus erfindungsgemäßer Verbindung, Lactose und Stärke wird mit einer 5%igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

**Oral applizierbare Lösung:**

Zusammensetzung:

500 mg der Verbindung von Beispiel 1, 2.5 g Polysorbat und 97 g Polyethylenglycol 400. Einer Einzeldosis von 100 mg der erfindungsgemäßen Verbindung entsprechen 20 g orale Lösung.

**Herstellung:**

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

**i.v. Lösung:**

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5%, PEG 400 Lösung 30%) gelöst. Die Lösung wird steril filtriert und in sterile und pyrogenfreie Injektionsbehältnisse abgefüllt.

## Patentansprüche

1. Verbindung der Formel

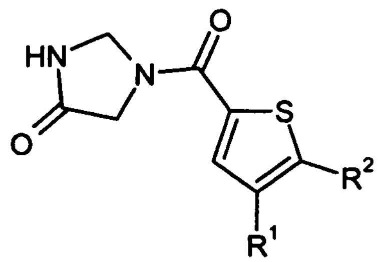

(I),

in welcher

$R^1$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1\text{-}C_4)$-Alkyl und $(C_1\text{-}C_4)$-Alkoxy,
worin
$(C_1\text{-}C_4)$-Alkyl und $(C_1\text{-}C_4)$-Alkoxy ihrerseits ein- bis dreifach, gleich oder verschieden, mit Resten substituiert sein können, die ausgewählt werden aus der Reihe Halogen, Cyano, Hydroxy, $(C_1\text{-}C_4)$-Alkoxy, Amino, Mono-$(C_1\text{-}C_4)$alkylamino, Di-$(C_1\text{-}C_4)$-alkylamino, $(C_3\text{-}C_7)$-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, $(C_1\text{-}C_4)$-Alkyl, Trifluormethyl, Hydroxy, $(C_1\text{-}C_4)$-Alkoxy, Trifluormethoxy, Oxo, Amino, Mono-$(C_1\text{-}C_4)$-alkylamino und Di-$(C_1\text{-}C_4)$-alkylamino substituiert sein können, und
$R^2$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1\text{-}C_4)$-Alkyl und $(C_1\text{-}C_4)$-Alkoxy,
worin
$(C_1\text{-}C_4)$-Alkyl und $(C_1\text{-}C_4)$-Alkoxy ihrerseits ein- bis dreifach, gleich oder verschieden, mit Resten substituiert sein können, die ausgewählt werden aus der Reihe Halogen, Cyano, Hydroxy, $(C_1\text{-}C_4)$-Alkoxy, Amino, Mono-$(C_1\text{-}C_4)$-alkylamino, Di-$(C_1\text{-}C_4)$-alkylamino, $(C_3\text{-}C_7)$-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, $(C_1\text{-}C_4)$-Alkyl, Trifluormethyl, Hydroxy, $(C_1\text{-}C_4)$-Alkoxy, Trifluormethoxy, Oxo, Amino, Mono-$(C_1\text{-}C_4)$-alkylamino und Di-$(C_1\text{-}C_4)$-alkylamino substituiert sein können,

oder eines ihrer Salze, ihrer Solvate oder der Solvate ihrer Salze.

2. Verbindung nach Anspruch 1, **dadurch gekennzeichnet, dass**

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1$-$C_4)$-Alkyl und $(C_1$-$C_4)$-Alkoxy, und
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1$-$C_4)$-Alkyl und $(C_1$-$C_4)$-Alkoxy,
worin
$(C_1$-$C_4)$-Alkoxy seinerseits ein- bis dreifach, gleich oder verschieden, mit Resten substituiert sein kann, die ausgewählt werden aus der Reihe Halogen, Cyano, Hydroxy, $(C_1$-$C_4)$-Alkoxy, Amino, Mono-$(C_1$-$C_4)$-alkylamino, Di-$(C_1$-$C_4)$-alkylamino, $(C_3$-$C_7)$-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, $(C_1$-$C_4)$-Alkyl, Trifluormethyl, Hydroxy, $(C_1$-$C_4)$-Alkoxy, Trifluormethoxy, Oxo, Amino, Mono-$(C_1$-$C_4)$-alkylamino und Di-$(C_1$-$C_4)$-alkylamino substituiert sein können,

oder eines ihrer Salze, ihrer Solvate oder der Solvate ihrer Salze.

3. Verbindung nach Anspruch 1 oder 2, **dadurch gekennzeichnet, dass**

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl, Methyl und Methoxy, und
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl, Trifluormethoxy, Methyl und $(C_1$-$C_3)$-Alkoxy,

oder eines ihrer Salze, ihrer Solvate oder der Solvate ihrer Salze.

4. Verbindung nach einem der Ansprüche 1 bis 3, **dadurch gekennzeichnet, dass** sie der Formel

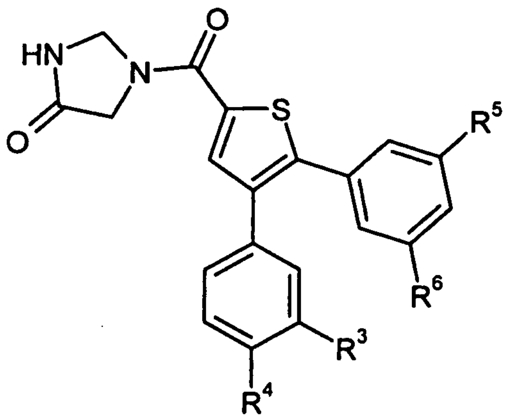

**(Ia),**

entspricht, in welcher

$R^3$ für Halogen oder Cyano steht,
$R^4$ für Wasserstoff oder Halogen steht,
$R^5$ für Halogen, Cyano oder Trifluormethyl steht, und
$R^6$ für Wasserstoff oder Halogen steht,

oder eines ihrer Salze, ihrer Solvate oder der Solvate ihrer Salze.

**5.** Verbindung nach Anspruch 4, **dadurch gekennzeichnet, dass**

$R^3$ für Fluor, Chlor oder Cyano steht,
$R^4$ für Wasserstoff, Chlor oder Fluor steht,
$R^5$ für Fluor, Chlor oder Cyano steht, und
$R^6$ für Wasserstoff, Chlor oder Fluor steht,

oder eines ihrer Salze, ihrer Solvate oder der Solvate ihrer Salze.

**6.** Verbindung nach Anspruch 4 oder 5, **dadurch gekennzeichnet, dass**

$R^3$ für Chlor oder Cyano steht,
$R^4$ für Wasserstoff oder Fluor steht,
$R^5$ für Chlor oder Cyano steht, und
$R^6$ für Wasserstoff oder Fluor steht,

oder eines ihrer Salze, ihrer Solvate oder der Solvate ihrer Salze.

**7.** Verfahren zur Herstellung einer Verbindung der Formel (I) nach Anspruch 1, **dadurch gekennzeichnet, dass** eine Verbindung der Formel

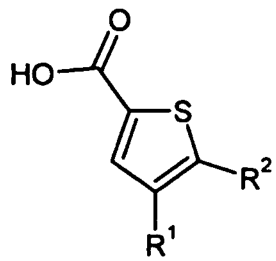

(II),

in welcher
$R^1$ und $R^2$ die in Anspruch 1 angegebene Bedeutung aufweisen,
mit Imidazolidin-4-on oder einem Salz von Imidazolidin-4-on umgesetzt wird.

**8.** Verfahren zur Herstellung einer Verbindung der Formel (I) nach Anspruch 1, **dadurch gekennzeichnet, dass** eine Verbindung der Formel

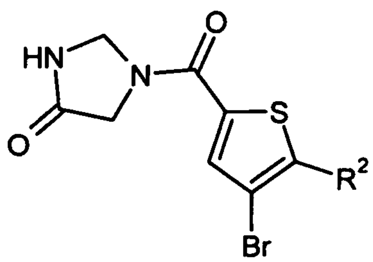

(VIII),

in welcher
$R^2$ die in Anspruch 1 angegebene Bedeutung aufweist,
unter Suzuki-Kupplungsbedingungen mit einer Verbindung der Formel

$R^1$-Q (V),

in welcher
$R^1$ die in Anspruch 1 angegebene Bedeutung aufweist und

Q für $-B(OH)_2$, einen Boronsäure-Ester, bevorzugt Boronsäurepinakolester, oder $-BF_3^-K^+$ steht,

umgesetzt wird.

**9.** Verfahren zur Herstellung einer Verbindung der Formel (I) nach Anspruch 1, **dadurch gekennzeichnet, dass** eine Verbindung der Formel

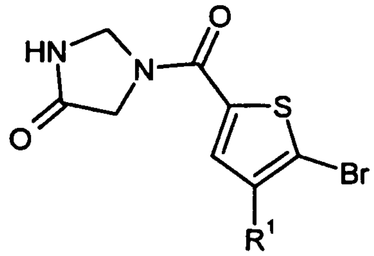

(X),

in welcher
$R^1$ die in Anspruch 1 angegebene Bedeutung aufweist,
unter Suzuki-Kupplungsbedingungen mit einer Verbindung der Formel

$R^7$-Q (VII),

in welcher
$R^2$ die in Anspruch 1 angegebene Bedeutung aufweist und

Q für $-B(OH)_2$, einen Boronsäure-Ester, bevorzugt Boronsäurepinakolester, oder $-BF_3^-K^+$ steht,

umgesetzt wird.

**10.** Verbindung nach einem der Ansprüche 1 bis 6 zur Behandlung und/oder Prophylaxe von Krankheiten.

**11.** Verwendung einer Verbindung nach einem der Ansprüche 1 bis 6 zur Herstellung eines Arzneimittels zur Behandlung und/oder Prophylaxe von Krankheiten.

**12.** Verwendung einer Verbindung nach einem der Ansprüche 1 bis 6 zur Herstellung eines Arzneimittels zur Behandlung und/oder Prophylaxe von retroviralen Erkrankungen.

**13.** Verwendung nach Anspruch 12, **dadurch gekennzeichnet, dass** die retrovirale Erkrankung eine Infektion mit dem HI-Virus ist.

**14.** Arzneimittel enthaltend mindestens eine Verbindung nach einem der Ansprüche 1 bis 6 in Kombination mit mindestens einem weiteren Wirkstoff.

**15.** Arzneimittel enthaltend mindestens eine Verbindung nach einem der Ansprüche 1 bis 6 in Kombination mit einem inerten nicht-toxischen pharmazeutisch geeigneten Hilfsstoff.

**16.** Arzneimittel nach Anspruch 14 oder 15 zur Behandlung und/oder Prophylaxe von retroviralen Erkrankungen.

**17.** Arzneimittel nach Anspruch 16, **dadurch gekennzeichnet, dass** die retrovirale Erkrankung eine Infektion mit dem HI-Virus ist.

## Claims

1. **1.** Compound of formula

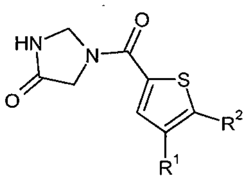

(I),

in which

$R^1$ represents phenyl,
whereby phenyl is substituted with 1 to 3 substituents, whereby the substituents are selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy,
wherein
$(C_1-C_4)$-alkyl and $(C_1-C_4)$-alkoxy in turn may be substituted one to three times identically or differently with radicals selected from the group consisting of halogen, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyl and 4- to 7-membered heterocyclyl,
whereby the last-mentioned cycloalkyl and heterocyclyl radicals in turn may each be substituted up to three times identically or differently with halogen, cyano, $(C_1-C_4)$-alkyl, trifluoromethyl, hydroxy, $(C_1-C_4)$-alkoxy, trifluoromethoxy, oxo, amino, mono-$(C_1-C_4)$-alkylamino and di-$(C_1-C_4)$-alkylamino, and
$R^2$ represents phenyl,
whereby phenyl is substituted with 1 to 3 substituents, whereby the substituents are selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy,
wherein
$(C_1-C_4)$-alkyl and $(C_1-C_4)$-alkoxy in turn may be substituted one to three times identically or differently with radicals selected from the group consisting of halogen, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyl and 4- to 7-membered heterocyclyl,
whereby the last-mentioned cycloalkyl and heterocyclyl radicals in turn may each be substituted up to three times identically or differently with halogen, cyano, $(C_1-C_4)$-alkyl, trifluoromethyl, hydroxy, $(C_1-C_4)$-alkoxy, trifluoromethoxy, oxo, amino, mono-$(C_1-C_4)$-alkylamino and di-$(C_1-C_4)$-alkylamino,

or one of the salts thereof, the solvates thereof or the solvates of the salts thereof.

2. **2.** Compound according to claim 1, **characterized in that**

$R^1$ represents phenyl,
whereby phenyl is substituted with 1 to 2 substituents, whereby the substituents are selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy, and
$R^2$ represents phenyl,
whereby phenyl is substituted with 1 to 2 substituents, whereby the substituents are selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy,
wherein
$(C_1-C_4)$-alkoxy in turn may be substituted one to three times identically or differently with radicals selected from the group consisting of halogen, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyl and 4- to 7-membered heterocyclyl,
whereby the last-mentioned cycloalkyl and heterocyclyl radicals in turn may each be substituted up to three times identically or differently with halogen, cyano, $(C_1-C_4)$-alkyl, trifluoromethyl, hydroxy, $(C_1-C_4)$-alkoxy, trifluoromethoxy, oxo, amino, mono-$(C_1-C_4)$-alkylamino and di-$(C_1-C_4)$-alkylamino,

or one of the salts thereof, the solvates thereof or the solvates of the salts thereof.

**3.** Compound according to claim 1 or 2, **characterized in that**

$R^1$ represents phenyl,
whereby phenyl is substituted with 1 to 2 substituents, whereby the substituents are selected independently of one another from the group consisting of halogen, cyano, trifluoromethyl, methyl and methoxy,
$R^2$ represents phenyl,
whereby phenyl is substituted with 1 to 2 substituents, whereby the substituents are selected independently of one another from the group consisting of halogen, cyano, trifluoromethyl, trifluoromethoxy, methyl and $(C_1-C_3)$-alkoxy,

or one of the salts thereof, the solvates thereof or the solvates of the salts thereof.

**4.** Compound according to any one of claims 1 to 3, **characterized in that** it corresponds to formula

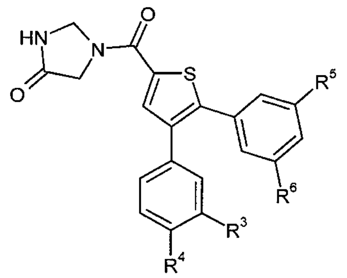

(Ia),

in which

$R^3$ represents halogen or cyano,
$R^4$ represents hydrogen or halogen,
$R^5$ represents halogen, cyano or trifluoromethyl, and
$R^6$ represents hydrogen or halogen,

or one of the salts thereof, the solvates thereof or the solvates of the salts thereof.

**5.** Compound according to claim 4, **characterized in that**

$R^3$ represents fluorine, chlorine or cyano,
$R^4$ represents hydrogen, chlorine or fluorine,
$R^5$ represents fluorine, chlorine or cyano, and
$R^6$ represents hydrogen, chlorine or fluorine,

or one of the salts thereof, the solvates thereof or the solvates of the salts thereof.

**6.** Compound according to claim 4 or 5, **characterized in that**

$R^3$ represents chlorine or cyano,
$R^4$ represents hydrogen or fluorine,
$R^5$ represents chlorine or cyano, and
$R^6$ represents hydrogen or fluorine,

or one of the salts thereof, the solvates thereof or the solvates of the salts thereof.

**7.** Method for preparing a compound of formula (I) according to claim 1, **characterized in that** a compound of formula

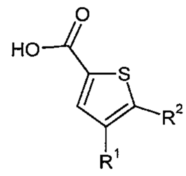

(II),

in which
$R^1$ and $R^2$ have the meaning given in claim 1,
is reacted with imidazolidin-4-one or with a salt of imidazolidin-4-one.

8. Method for preparing a compound of formula (I) according to claim 1, **characterized in that** a compound of formula

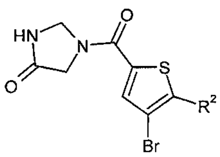

(VIII),

in which
$R^2$ has the meaning given in claim 1,
is reacted under Suzuki coupling conditions with a compound of formula

$R^1$-Q (V),

in which
$R^1$ has the meaning given in claim 1 and

Q represents $-B(OH)_2$, a boronic acid ester, preferably boronic acid pinacol ester, or $-BF_3^-K^+$.

9. Method for preparing a compound of formula (I) according to claim 1, **characterized in that** a compound of formula

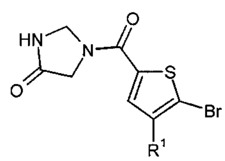

(X),

in which
$R^1$ has the meaning given in claim 1,
is reacted under Suzuki coupling conditions with a compound of formula

$R^2$-Q (VII),

in which
$R^2$ has the meaning given in claim 1 and

Q represents $-B(OH)_2$, a boronic acid ester, preferably boronic acid pinacol ester, or $-BF_3^-K^+$.

10. Compound according to any one of claims 1 to 6 for the treatment and/or prophylaxis of diseases.

11. Use of a compound according to any one of claims 1 to 6 for the manufacture of a medicament for the treatment and/or prophylaxis of diseases.

12. Use of a compound according to any one of claims 1 to 6 for the manufacture of a medicament for the treatment

and/or prophylaxis of retroviral diseases.

**13.** Use according to claim 12, **characterized in that** the retroviral disease is an infection with the HI virus.

**14.** Medicament comprising at least one compound according to any one of claims 1 to 6 in combination with at least one further active compound.

**15.** Medicament comprising at least one compound according to any one of claims 1 to 6 in combination with at least one inert, non-toxic, pharmaceutically acceptable excipient.

**16.** Medicament according to claim 14 or 15 for the treatment and/or prophylaxis of retroviral diseases.

**17.** Medicament according to claim 16, **characterized in that** the retroviral disease is an infection with the HI virus.

## Revendications

**1.** Composé de formule

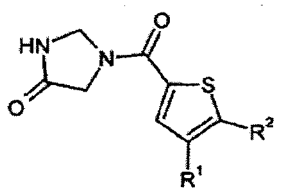
(I),

dans laquelle
$R^1$ désigne un groupe phényle
le groupe phényle étant substitué par 1 à 3 substituants, les substituants étant choisis indépendamment les uns des autres dans le groupe comprenant un atome d'halogène, un groupe hydroxy, cyano, nitro, trifluorométhyle, trifluorométhoxy, trifluorométhylthio, alkyle (en $C_1$ à $C_4$) et alcoxy (en $C_1$ à $C_4$),
où
les groupes alkyle (en $C_1$ à $C_4$) et alcoxy en ($C_1$ à $C_4$) peuvent de leur côté être mono à trisubstitués, de façon identique ou différente, par des radicaux choisis dans la série comprenant un atome d'halogène, un groupe cyano, hydroxy, alcoxy (en $C_1$ à $C_4$), amino, mono-alkylamino (en $C_1$ à $C_4$), di-alkylamino (en $C_1$ à $C_4$), cycloalkyle (en $C_3$ à $C_7$) et hétérocyclyle de 4 à 7 chaînons,
les radicaux cycloalkyle et hétérocyclyle cités en dernier lieu pouvant de leur côté être substitués jusqu'à trois fois, de façon identique ou différente, par un atome d'halogène, un groupe cyano, alkyle (en $C_1$ à $C_4$), trifluorométhyle, hydroxy, alcoxy (en $C_1$ à $C_4$), trifluorométhoxy, oxo, amino, mono-alkylamino (en $C_1$ à $C_4$) et di-alkylamino (en $C_1$ à $C_4$), et
$R^2$ désigne un groupe phényle,
le groupe phényle étant substitué par 1 à 3 substituants, les substituants étant choisis indépendamment les uns des autres dans le groupe comprenant un atome d'halogène, un groupe hydroxy, cyano, nitro, trifluorométhyle, trifluorométhoxy, trifluorométhylthio, alkyle (en $C_1$ à $C_4$) et alcoxy (en $C_1$ à $C_4$),
où
les groupes alkyle (en $C_1$ à $C_4$) et alcoxy en ($C_1$ à $C_4$) peuvent de leur côté être mono à trisubstitués, de façon identique ou différente, par des radicaux choisis dans la série comprenant un atome d'halogène, un groupe cyano, hydroxy, alcoxy (en $C_1$ à $C_4$), amino, mono-alkylamino (en $C_1$ à $C_4$), di-alkylamino (en $C_1$ à $C_4$), cycloalkyle (en $C_3$ à $C_7$) et hétérocyclyle de 4 à 7 chaînons,
les radicaux cycloalkyle et hétérocyclyle cités en dernier lieu pouvant de leur côté être substitués jusqu'à trois fois, de façon identique ou différente, par un atome d'halogène, un groupe cyano, alkyle (en $C_1$ à $C_4$), trifluorométhyle, hydroxy, alcoxy (en $C_1$ à $C_4$), trifluorométhoxy, oxo, amino, mono-alkyleamino (en $C_1$ à $C_4$) et di-alkylamino (en $C_1$ à $C_4$).
ou l'un de ses sels, de ses solvates ou des solvates de ses sels.

**2.** Composé selon la revendication 1, **caractérisé en ce que**

$R^1$ désigne un groupe phényle,
le groupe phényle étant substitué par 1 à 2 substituants, les substituants étant choisis indépendamment les uns des autres dans le groupe comprenant un atome d'halogène, un groupe hydroxy, cyano, nitro, trifluorométhyle, trifluorométhoxy, trifluorométhylthio, alkyle (en $C_1$ à $C_4$) et alcoxy (en $C_1$ à $C_4$), et
$R^2$ désigne un groupe phényle,
le groupe phényle étant substitué par 1 à 2 substituants, les substituants étant choisis indépendamment les uns des autres dans le groupe comprenant un atome d'halogène, un groupe hydroxy, cyano, nitro, trifluorométhyle, trifluorométhoxy, trifluorométhylthio, alkyle (en $C_1$ à $C_4$) et alcoxy (en Ci à $C_4$),
où
le groupe alcoxy en ($C_1$ à $C_4$) peut de son côté être mono à trisubstitué, de façon identique ou différente, par des radicaux choisis dans la série comprenant un atome d'halogène, un groupe cyano, hydroxy, alcoxy (en $C_1$ à $C_4$), amino, mono-alkylamino (en $C_1$ à $C_4$), di-alkylamino (en $C_1$ à $C_4$), cycloalkyle (en $C_3$ à $C_7$) et hétérocyclyle de 4 à 7 chaînons,
les radicaux cycloalkyle et hétérocyclyle cités en dernier lieu pouvant de leur côté être substitués jusqu'à trois fois, de façon identique ou différente, par un atome d'halogène, un groupe cyano, alkyle (en $C_1$ à $C_4$), trifluorométhyle, hydroxy, alcoxy (en $C_1$ à $C_4$), trifluorométhoxy, oxo, amino, mono-alkyle (en $C_1$ à $C_4$)-amino et di-alkylamino (en $C_1$ à $C_4$),
ou l'un de ses sels, de ses solvates ou des solvates de ses sels.

3. Composé selon la revendication 1 ou 2, **caractérisé en ce que**
$R^1$ désigne un groupe phényle,
le groupe phényle étant substitué par 1 à 2 substituants, les substituants étant choisis indépendamment les uns des autres dans le groupe comprenant un atome d'halogène, un groupe cyano, trifluorométhyle, méthyle et méthoxy, et
$R^2$ désigne un groupe phényle,
le groupe phényle étant substitué par 1 à 2 substituants, les substituants étant choisis indépendamment les uns des autres dans le groupe comprenant un atome d'halogène, un groupe cyano, trifluorométhyle, trifluorométhoxy, méthyle et alcoxy (en $C_1$ à $C_3$),
ou l'un de ses sels, de ses solvates ou des solvates de ses sels.

4. Composé selon l'une des revendications 1 à 3, **caractérisé en ce qu'**il correspond à la formule

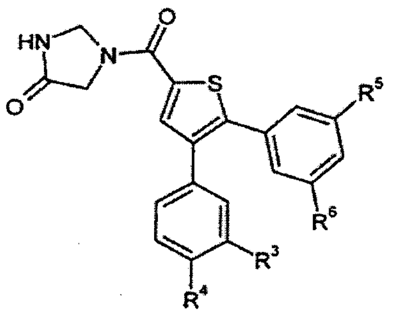

**(Ia),**

dans laquelle
$R^3$ désigne un atome d'halogène ou un groupe cyano,
$R^4$ désigne un atome d'hydrogène ou d'halogène,
$R^5$ désigne un atome d'halogène, un groupe cyano ou trifluorométhyle, et
$R^6$ désigne un atome d'hydrogène ou d'halogène,
ou fun de ses sels, de ses solvates ou des solvates de ses sels.

5. Composé selon la revendication 4, **caractérisé en ce que**
$R^3$ désigne un atome de fluor, de chlore ou un groupe cyano,
$R^4$ désigne un atome d'hydrogène, de chlore ou de fluor,
$R^5$ désigne un atome de fluor, de chlore ou un groupe cyano, et
$R^6$ désigne un atome d'hydrogène, de chlore ou de fluor,
ou l'un de ses sels, de ses solvates ou des solvates de ses sels.

**6.** Composé selon la revendication 4 ou 5, **caractérisé en ce que**
$R^3$ désigne un atome de chlore ou un groupe cyano,
$R^4$ désigne un atome d'hydrogène ou de fluor,
$R^5$ désigne un atome de chlore ou un groupe cyano, et
$R^6$ désigne un atome d'hydrogène ou de fluor,
ou l'un de ses sels, de ses solvates ou des solvates de ses sels.

**7.** Procédé de production d'un composé de formule (I) selon la revendication 1, **caractérisé en ce que** l'on fait réagir un composé de formule

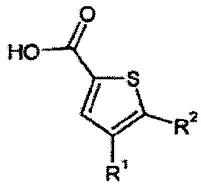
(II),

dans laquelle
$R^1$ et $R^2$ présentent la signification indiquée dans la revendication 1, avec de l'imidazolidin-4-one ou un sel d'imidazolidin-4-one.

**8.** Procédé de production d'un composé de formule (I) selon la revendication 1, **caractérisé en ce que** l'on fait réagir un composé de formule

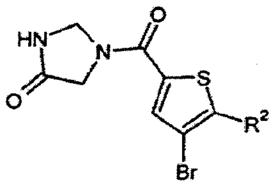
(VIII),

dans laquelle
$R^2$ présente la signification indiquée dans la revendication 1,
dans des conditions de couplage de Suzuki, avec un composé de formule

$R^1$-Q (V),

dans laquelle
$R^1$ présente la signification indiquée dans la revendication 1 et
Q désigne $-B(OH)_2$, un ester d'acide boronique, de préférence, un ester pinacolique d'acide boronique ou $-BF_3^-K^+$.

**9.** Procédé de production d'un composé de formule (I) selon la revendication 1, **caractérisé en ce que** l'on fait réagir un composé de formule

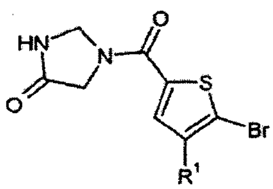
(X),

dans laquelle
$R^1$ présente la signification indiquée dans la revendication 1,
dans des conditions de couplage de Suzuki, avec un composé de formule

$R^2$-Q (VII),

dans laquelle
$R^2$ présente la signification indiquée dans la revendication 1 et
Q désigne -$B(OH)_2$, un ester d'acide boronique, de préférence, un ester pinacolique d'acide boronique ou -$BF_3^-K^+$.

**10.** Composé selon l'une des revendications 1 à 6, pour le traitement et/ou la prophylaxie de maladies.

**11.** Utilisation d'un composé selon l'une des revendications 1 à 6, pour la production d'un médicament pour le traitement et/ou la prophylaxie de maladies.

**12.** Utilisation d'un composé selon l'une des revendications 1 à 6, pour la production d'un médicament pour le traitement et/ou la prophylaxie de maladies rétrovirales.

**13.** Utilisation selon la revendication 12, **caractérisé en ce que** la maladie rétrovirale est une infection au VIH.

**14.** Médicament contenant au moins un composé selon l'une des revendications 1 à 6 en combinaison avec au moins une autre substance active.

**15.** Médicament contenant au moins un composé selon l'une des revendications 1 à 6, en combinaison avec un adjuvant inerte approprié, pharmaceutiquement non toxique.

**16.** Médicament selon la revendication 14 ou 15, pour le traitement et/ou la prophylaxie de maladies rétrovirales.

**17.** Médicament selon la revendication 16, **caractérisé en ce que** la maladie rétrovirale est une infection au VIH.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9119708 A1
- EP 0065295 A1
- WO 9427979 A1
- WO 0200649 A1
- WO 2006062982 A2
- WO 2006062984 A2
- US 5627203 A
- WO 03014107 A1
- US 20040116425 A1
- WO 2004016592 A1
- WO 2005035488 A2
- WO 2006023462 A1
- WO 20080903820 A1
- GB 453061 A


### In der Beschreibung aufgeführte Nicht-Patentliteratur


- **PALELLA et al.** *N. Engl. J. Med.,* 1998, vol. 238, 853-860
- **FLEXNER.** *Nature Reviews Drug Discovery,* 2007, vol. 6, 959-966
- **CARPENTER et al.** *J. Am. Med. Assoc.,* 2000, vol. 283, 381-390
- **FINZI et al.** *Nature Med.,* 1999, vol. 5, 512-517
- **RAMRATNAM et al.** *Nature Med.,* 2000, vol. 6, 82-85
- **KAVLICK ; MITSUYA.** Antiretroviral Chemotherapy. ASM Press, 2001, 279-312
- **ROMERO et al.** *J. Med. Chem,* 1994, vol. 37, 999-1014
- **HOUSTON JB.** Utility of in-vitro drug-metabolism data in predicting in-vivo metabolic-clearance. *Bioch. Pharm.,* 1994, vol. 47 (9), 1469-1479
- **OBACH RS ; BAXTER JG ; LISTON TE ; SILBER BM ; JONES BC ; MACINTYRE F ; RANCE DJ ; WASTALL P.** The prediction of human pharmacokinetic parameters from preclinical and in vitro metabolism data. *J. Pharmacol. Exp. Ther.,* 1997, vol. 283 (1), 46-58
- **PANG KS ; ROWLAND M.** Hepatic clearance of drugs. I. Theoretical considerations of a ''well-stirred'' model and a ''parallel tube'' model. Influence of hepatic blood flow, plasma and blood cell binding, and the hepatocellular enzymatic activity on hepatic drug clearance. *J Pharmacokinet Biopharm,* 1977, vol. 5 (6), 625-53